# EUROPEAN PATENT APPLICATION

(11) **EP 4 491 099 A1**
(43) Date of publication of application: **15.01.2025**
(21) Application number: 24188417.0
(22) Date of filing: 12.07.2024
(51) Int. Cl.: A61B 3/10, A61B 3/103, A61B 3/107, A61B 3/11, G02B 27/00, G02B 27/01, G02C 7/08

(54) **SMART EYEWEAR VISION CORRECTION AND ADJUSTMENT METHOD AND SYSTEM**

(30) Priority: 12.07.2023 US 202363513265 P
(71) Applicant: Plenoptika, Inc., Cambridge, MA 02139 (US)
(72) Inventor: DAVE, Shivang R., Boston, MA 02118 (US); LIM, Daryl, 589473 Singapore (SG)
(74) Representative: Høiberg P/S

(57) **Abstract**

Open view smart eyewear is combined with a wavefront aberrometer module. The smart eyewear serves as an open view eyewear form factor and house the wavefront aberrometer module among other digital processing components and corresponding software. Alternatively, the wavefront aberrometer module is removably coupled to the smart eyewear. The wavefront aberrometer module calibrates a tunable optical element according to detected optical needs of the user. The wavefront aberrometer module automatically adjusts the wearer-user's effective visual acuity (at least refractive power) viewing through the smart eyewear. The smart eyewear apparatus can also provide clinical-quality optical measurements of wearer-users for electronic communication or transmission to an eye care professional. Alignment of open view smart eyewear, the wavefront aberrometer module, and user's eyes (line of sight) can be facilitated. Iris biometric identification can be used to associate the user-specific calibration parameters and measurements with the smart eyewear.

## Description

### RELATED APPLICATIONS

This application claims the benefit of U.S. Provisional Application No. 63/513,265, filed on July 12, 2023.

This application is a continuation-in-part of U.S. Application No. 18/559,027, filed November 3, 2023, which is the U.S. National Stage of International Application No. PCT/US2022/072186, filed on May 6, 2022, published in English on November 10, 2022 as WO 2022/236333, which claims the benefit of U.S. Provisional Application No. 63/185,158, filed on May 6, 2021.

The entire teachings of the above Applications are incorporated herein by reference.

### GOVERNMENT SUPPORT

This invention was made with government support under R44 EY025452 from the National Institutes of Health. The government has certain rights in the invention.

### BACKGROUND

There is an increasing number of head-mounted viewing devices or portable wearable vision systems in use for personal entertainment, workplace applications, or other purposes, each without regard to vision correction. For example, there are head-mounted devices for virtual reality (VR) or augmented reality (AR) viewing, and there are smart eyewear with AR capabilities. In the first or early generations of these devices, none are directed to vision correction or adjusting visual acuity (especially objective refractive error) of the user. Instead the devices are intended to be worn or used in combination with prescription (corrective) eyewear of the user.

In more recent times, vision correction in smart eyewear is being considered. For example, US patent application Publication No. US20200174284 A1 entitled "Head-Mounted Display Device With Vision Correction" describes improvements desired for head-mounted devices such as VR and AR glasses. The disclosure states that such devices use displays to generate images and use lenses to focus the images on to the eyes of a user. Continuing, the disclosure states that extended use of a head-mounted device may become uncomfortable because the optical systems therein are bulky, heavy, and typically not sufficiently adjustable. Thus the head-mounted device becomes tiring to wear. The '284 publication discloses a sensor incorporated into a subject head-mounted device that measures refractive errors in the user's eyes. The device can enhance viewing comfort: (i) by adjusting display position relative to the user's eye positions, (ii) by adjusting lens settings based on the content being presented on the display, and/or (iii) based on measured eye refractive errors. The effect of the refractive errors of the user's vision such as far-sightedness, near-sightedness, and astigmatism may then be corrected by tuning the lenses and/or adjusting display positions.

Another possible cause of eyestrain mentioned in the '284 publication is accomodative-vergence mismatch. The subject device is disclosed to perform operations that lead to relaxing the user's ciliary muscles in turn minimizing eyestrain. For example, control circuitry periodically (e.g., every 20 minutes) presents to the user content at an apparent distance of at least 20 feet away and directs the user to look at this distant content for a predetermined amount of time. Further mentioned are adjustments to the diopter correction or other optical system settings associated with the subject device to improve user eye comfort. In the example given, the device is calibrated during manufacturing so that control circuitry places the display and optical system in a low-eye-strain configuration during normal operation for the user. When calibrating the device, the device determines the position of display that corresponds to a virtual image at infinity focus. The calibration information is then stored in the control circuitry.

European patent application no. EP3865046 discloses detecting and correcting: (i) a variation of current refractive error, and (ii) a variation of current accommodation amplitude of a person. In the aging of the eye, close objects appear blurry because the eye tends to focus light behind, instead of on, the retina. This is known as insufficient accommodation amplitude, and the ability to focus clearly on close objects progressively worsens. The EP application discloses methods for driving an active refractive lens for correcting a refractive error or correcting an accommodation amplitude of a person, while accounting for an evolution of the refractive error over time. The disclosed method and device include a continuous recording of changes in the refraction of the eyes of the given person and obtains statistics of accommodation response to inform about potential accommodation lag in near vision.

WO2022032198 A1 entitled "Tunable Cylindrical Lenses and Head-Mounted Display Including the Same" by MagicLeap discloses custom inserts in wearable AR display systems for users with non-emmetropic vision, such as short sighted (myopic) or far sighted (hyperopic) users. The custom inserts correct for a user's refractive error, e.g., according to ophthalmic prescription (Rx). Alternatively, the wearable display system can be designed to accommodate user-supplied prescription eyeglasses between the wearer and the display system's eyepiece. However, customization of a headset is both time-consuming and expensive. The MagicLeap display system may also include an eye-tracking module that requires biometric data of the user and depth-of fixation data. The eye-tracking module may communicate the biometric data (such as for iris identification purposes) and depth-of-fixation data to a local processor.

WO2022009233 A1 entitled "Apparatus and Method for Self-Correcting Objective Refractometry" by Forus Health Pvt. Ltd. discloses conducting vision tests on a user while the user is wearing a head mounted unit. The disclosed approach attaches a detachable optical unit to the head mounted unit. In turn, with the detachable optical unit attached, tests such as an objective refraction test, a subjective refraction test, field of vision test, color vision test, and so on are conducted on the user. Once the tests are completed and there is a subsequent confirmation that the first computed refraction error in the user's vision is corrected, the optical unit is detached or rotated away (see Figure 3 of WO2022009233). As a result, the user is able to view a scene through a tunable lens and the error in vision is compensated for.

### SUMMARY

Lacking in the art are methods and systems for correcting objective refraction error of users of open-view smart eyewear, e.g., Augmented Reality headsets, Virtual Reality headsets, and Mixed Reality headsets. Applicants address these shortcomings and problems long existing in the art. In particular, embodiments of the present invention provide objective measurements of the user's refractive power. In this way, Applicant's approach addresses the need to: (i) correct pre-existing refractive errors in user vision; (ii) track change in the user's refractive power during use of the smart eyewear, for non-limiting example, due to the user looking at objects close and then far, or vice versa; (iii) track change in the user's accommodative response (or lag), or changes in accommodative amplitude; and/or (iv) reduce eyestrain and increase user comfort and period of useability of the smart eyewear.

Some embodiments further include a biometric identification of the user associating so identified user with the device (for security) and with device settings (for customized calibration).

Other embodiments additionally enable generation of or support of a healthcare professional to generate an eyeglass prescription for corrective lenses for the user.

So-called smart eyewear are wearable digital processing technology in the form factor of eyewear, e.g., eyeglasses, sunglasses, monocles, goggles, and the like. The eyewear apparatus has internal components that digitally process information and render or otherwise present results (such as visually display image content on a lens surface of the eyewear or on a separate micro display screen, and/or audibly play sound through a speaker) to the wearer-user. The resulting visual display may be Virtual Reality, Augmented Reality, or Modified Reality. The internal electronic components may include elements that provide internet connectivity, network level communications, etc. In turn, such connectivity and communications can support real-time features and software program application use.

Embodiments of the present invention combine a working wavefront aberrometer module with smart eyewear to form an open view eyewear apparatus or system that automatically adjusts objective refractive error of the wearer-user while looking through the smart eyewear at multiple distances and while looking down the optical center. That is, a pair of smart glasses (or a head worn processor device in the form of eyewear with an open view) is programmed or otherwise configured with wavefront aberrometer sensors, functions and associated operations that determine optical properties of the eye. The functions, operations, and techniques implemented or performed by the wavefront aberrometer module are generally as described in PCT International Application no. PCT/US2022/072186 filed on May 6, 2022, published in English on November 10, 2022 as WO 2022/236333, and herein incorporated by reference in its entirety. Applicants herein provide a new use of the wavefront aberrometer, namely as a calibration mechanism for smart eyewear.

In some embodiments, the working wavefront aberrometer module is removably attachable to the smart eyewear (pair of open view smart glasses) and serves as a tool by a technician at a point of sale, service center, and the like for non-limiting example. In this mode, the technician uses or applies the working wavefront aberrometer module as a calibration tool configuring the tunable lens or optic elements of the smart eyewear specifically for the user. In this way, the embodiment system/apparatus custom tunes for multiple depths, multiple lighting conditions, and target contrast (generally herein multiple "viewing conditions"). The resulting custom calibration parameters and related information are stored in local memory of the smart eyewear, cloud memory in communication with the smart eyewear, or the like.

In another embodiment, the working wavefront aberrometer module is configured for removable attachment to the smart eyewear (pair of open view smart glasses) by the user and serves as an optional mobile app accessory of sorts. In this mode, the user temporarily applies the working wavefront aberrometer module to personalize (calibrate and configure through the mobile app) the tunable optics of the smart eyewear before, during, and/or after use (i.e., between uses). In this mode, the embodiment system/apparatus, i.e., the combined working wavefront aberrometer module and smart eyewear with open view form factor, dynamically and continuously applies Applicant's wavefront aberrometry techniques during user use of the system/apparatus, and responsively adjusts the tunable optics and/or display of the smart eyewear while the user is viewing objects at different depths, lighting conditions, and/or target contrast. In this way, the embodiment system/apparatus custom tunes the smart eyewear for multiple viewing conditions (i.e., depths, light conditions, and/or target contrast). The resulting custom calibration parameters and related information are stored in local memory of the smart eyewear, mobile app file storage, cloud memory in communication with the mobile app or smart eyewear, or the like.

In yet another embodiment, the working wavefront aberrometer module is built in and coupled with the internal components of the smart eyewear. In this mode, the embodiment system/apparatus, i.e., the combined working wavefront aberrometer module and smart eyewear with open view form factor, dynamically and continuously applies Applicant's wavefront aberrometry techniques during user use of the system/apparatus, and responsively adjusts the tunable optics and/or display of the smart eyewear while the user is viewing objects at different depths. In this way, the embodiment system/apparatus custom tunes the smart eyewear for multiple depths, multiple lighting conditions, and/or multiple target contrast (i.e., multiple viewing conditions).

In embodiments, the working wavefront aberrometer module assesses the user's objective refraction and adjusts the wearer-user's visual acuity (or at least corrects objective refractive error) while viewing through the smart eyewear by updating the tunable optics within the smart eyewear. The working wavefront aberrometer module communicates measured objective refraction or corrective vision prescription information to the controller of the smart eyewear processor. In response, the controller processor updates the tunable optics which in turn modify the contents displayed in a manner that is specific to the user's refractive power/sensed vision performance of the user and that improves the user's visual experience and comfort. Or for smart eyewear that does not locally employ tunable optics (lens subsystem), the controller processor responsively modifies contents displayed to the user in a manner that is specific to the sensed refractive power/sensed vision of the user and that improves the user's visual experience and comfort. The working wavefront aberrometer module in near real time and continuously assesses the user's objective refraction and automatically corrects the user's objective refractive error viewing through the smart eyewear by so adjusting and updating the tunable optics and/or resulting display to the user. In this way, the working wavefront aberrometer module also calibrates the smart glasses for use by the specific user.

Thus, embodiments of the present invention provide advantages and efficiencies lacking in the art. In particular, Applicants address long felt needs pertaining to: (i) assessing the refractive state of the user (at a single depth of focus or multiple, and under standard or multiple lighting conditions) and using that information to adjust the visual acuity (specifically, refractive power and/or contrast sensitivity) of a wearer-user of open view head-mounted viewing devices, smart glasses, smart eyewear, portable head-mounted apparatus, and the like, and (ii) assessing the tunable optics or providing the necessary information required for the smart eyewear (its controller and optics/display subsystems) to update the content displayed to the wearer-user to improve visual experience and/or comfort. The assessing of refractive state of the user is either ongoing during use of the smart eyewear or as an initial calibration to configure the smart eyewear according to the specific user's visual needs and ability (accommodative power/amplitude, and/or accommodative lag).

In embodiments, the digital processing components of the smart eyewear and/or the working wavefront aberrometer module include a pupil camera and corresponding executable code. The corresponding executable code is configured to assist in aligning, or in maintaining alignment of, one or more optical elements of the smart eyewear system (wavefront aberrometer module) with a pupil of an eye of the user. Maintaining alignment includes maintaining for a sufficient time (3 seconds, 5 seconds, 10 seconds, 20 seconds, or 30 seconds for non-limiting example) for the working wavefront aberrometer module to acquire a temporal sequence of wavefront aberrometry measurements of the eye. In embodiments, maintaining alignment includes maintaining for a sufficient time to obtain a set of objective refractive error measurements of the user while the user views one or more external target indicia at multiple depths or with multiple ambient light (and contrast) conditions through the optical center (or otherwise) of at least one tunable optical element of the smart eyewear system.

In some embodiments, the open view smart eyewear apparatus or system includes a working wavefront aberrometer module and is of a binocular or monocular, open field design. The working wavefront aberrometer module can include a tunable-lens based phoropter system, pupil camera, keratometer, retinal imaging capability, and other features. Non-limiting examples of such portable optical devices are described in: US Application Publication no. 2020/0046222 by Shivang R. Dave et al.*,* US Patent no. 9,854,965 by Nicholas J. Durr et al.*,* and US Patent no. 10,786,150 by Nicholas J. Durr et al.*,* each incorporated herein in their entireties.

In some embodiments, corresponding software uploads optical measurement data generated by the open view smart eyewear apparatus to a physician/health professional or artificial intelligence server. The corresponding software thus provides uploading and livestreaming of the optical measurement data (generated by the working wavefront aberrometer module) to the physician/health professional or artificial intelligence server as a function of type of measurement data and/or health condition of the wearer-user. In turn, the health professional may arrive at or have sufficient information to generate a prescription for general use, non-smart eyewear, corrective lenses for the user. Such an embodiment addresses the need for conducting remote, out of clinical setting, eye exams to a population of users.

### BRIEF DESCRIPTION OF THE DRAWINGS

The foregoing will be apparent from the following more particular description of example embodiments, as illustrated in the accompanying drawings in which like reference characters refer to the same parts throughout the different views. The drawings are not necessarily to scale, emphasis instead being placed upon illustrating embodiments.
FIG. 1 (prior art) is a schematic block diagram illustrating an embodiment apparatus for determining a property of an eye.
FIG. 2 (prior art) is a schematic block diagram illustrating an alternative embodiment apparatus that is open view and also includes other optional features.
FIG. 3 (prior art) is a schematic diagram illustrating various optional input and output features of embodiment devices, such as those illustrated in FIGs. 1-2.
FIG. 4 (prior art) is a computer interconnect diagram illustrating various components of the determination and control module in FIG. 2 and its connections to various components, including some components illustrated in FIG. 2, some optional components shown in FIG. 3, as well as some that are not illustrated in FIGs. 2-3.
FIG. 5A (prior art) is a top-view illustration of subject, binocular, wavefront aberrometer autorefractor apparatus with a lensometer module attached; the apparatus of FIG. 5A is also referred to as "QuickSee" apparatus herein.
FIGs. 5B-5C (prior art) are side-view illustrations of the apparatus illustrated in FIG. 5A. FIG. 5B shows eyeglasses outside of the lensometer module, while FIG. 5C shows the eyeglasses inserted into the lensometer attachment.
FIG. 6A (prior art) is a flow diagram illustrating generally how embodiment devices and methods can be used to perform objective refraction measurements.
FIG. 6B (prior art) is a flow diagram illustrating generally how embodiment devices and methods can be used to perform subjective refractive measurements.
FIG. 6C (prior art) is a flow diagram illustrating generally how embodiment devices and methods can be used to measure accommodation amplitude for evaluation of presbyopia.
FIG. 7 (prior art) is a flow diagram illustrating an embodiment procedure for determining a property of an eye.
FIG. 8 is a schematic diagram illustrating a portable optical device, such as smart eyewear, that includes a pupil camera, consistent with various embodiments.
FIG. 9 is a pair of images of a human pupil imaged with a device that is similar to that of FIG. 8 having a pupil camera.
FIGs. 10A-10B (prior art) are flow diagrams illustrating parts of a general procedure for determining subjective refractive measurements (phoroptry) for refractive eye correction using subject wavefront aberrometer devices directly interacting with a patient.
FIG. 11 is a series of three keratometry images illustrating how keratometry data can be derived by using a pupil camera such as the one described in FIG. 8 according to embodiments.
FIG. 12 is a flow diagram illustrating a procedure for alignment of device optics to a patient's pupil according to embodiments.
FIG. 13 is a flow diagram illustrating an example process for obtaining various eye health parameters from portable optical devices according to embodiments devices, systems, and methods.
FIG. 14 is a flow diagram illustrating how pupil camera images can be processed in order to determine position of the pupil and provide alignment feedback according to embodiments.
FIG. 15 is a flow diagram illustrating how a sequence of images captured by a pupil camera on a portable optical device can be processed to obtain keratometry values for an eye.
FIG. 16 is a schematic diagram of a cloud environment interconnecting a patient and portable optical device with other entities.
FIG. 17 is a schematic plan view of a smart eyewear system embodying the present invention.
FIG. 18A and 18B are illustrations of an open view smart eyewear system or apparatus embodying principles of the present invention.
FIG. 19 is a schematic diagram of the smart eyewear apparatus of FIG. 18B.
FIG. 20 is a flow diagram of calibrating a pair of open view smart eyewear in one embodiment.
FIG. 21 is a flow diagram of adjusting tunable optical elements with a working wavefront aberrometer module installed on a pair of open view smart eyewear in embodiments.
FIG. 22 is a flow diagram of adjusting tunable optical elements after calibration by and removal of the working wavefront aberrometer module of embodiments.

### DETAILED DESCRIPTION

A description of example embodiments follows.

As used herein, the terms "patient," "wearer-user," and "user" are synonymous and interchangeable. The terms "portable optical device," "smart glasses apparatus," "smart glasses system," " smart eyewear," "AR headset," "VR headset," and "MR headset," are used interchangeably. The corresponding software program(s) may be provided to the wearer-user: preloaded on the smart eyewear apparatus, via a cloud server, through a website, or through an online platform accessed by the smart eyewear apparatus. Further "VR" Virtual Reality generally refers to a computer generated or simulated three-dimensional environment (images, sounds, sensations) that provides an immersive and interactive experience to the user through near eye displays. Generally, "AR" Augmented Reality is a user interactive experience like Virtual Reality but combines real world and virtual world content. In AR, computer generated display portions overlay additively to or mask out from the current real (natural) environment of the user. Restated, augmented reality alters the user's perception of the current real-world environment (and does not keep the user immersed) whereas virtual reality completely replaces the user's real-world environment with a computer generated and simulated one. "MR" Mixed Reality is a blend of physical and digital worlds forming a hybrid environment having natural and intuitive human-computer-environment interactions in three dimensions. In MR, virtual objects interact with physical world objects. That is, Mixed Reality is an extension of Augmented Reality where real-world elements and virtual elements interact in an environment. MR is also known as XR "Extended Reality."

Wavefront aberrometry is an objective method of measuring the manner in which the wavefront generated by a light beam is distorted as it moves through an optical system such as a tested eye. The method offers detailed measurements of the light wavefront. The detailed measurements can be used in diagnosis of both higher-order and lower-order refractive errors of the eye and can also be used to assess other vision-impacting diseases of the eye. A wavefront aberrometer is a tool that employs the forgoing wavefront technology to measure how the wavefront generated by a light beam is distorted as it travels through a tested eye. If the tested eye has visual errors, the wavefront takes on different shapes that can be used to define those errors.

Visual acuity is a measure of how well one can see small details with precision or generally a measure of clarity of vision of an eye. Visual acuity depends on two basic categories of factors: optical factors (refractive power, health, and functioning) of the eye, and neural factors (health and functioning in pertinent parts) of the brain. For example, common optical causes of low visual acuity include refractive errors, that is, errors in how the light is refracted in the eyeball. Causes of refractive errors include aberrations in the shape, length, curvature, and thickness of the eyeball or the cornea, other corneal irregularities, and reduced ability of the lens of the eye to focus light. In other examples, neural factors, or cells and tissue of the retina limit visual acuity. Visual acuity may be limited by compromised health and functioning of the retina, of the neural pathways to the brain, and of the interpretive faculty of the brain. A visual acuity measurement of a tested eye has an objective component (e.g., objective refractive error) and a subjective component (e.g., person's stated preferences).

An autorefractor is a computer-controlled machine used during an eye examination to provide an objective measurement of an eye's refractive error. The machine measures how light is changed as it enters and exits the eye, specifically sensors in the machine detect reflections of the (typically infrared) light that entered and exited the eye. The machine uses these reflections to determine the size and shape of a ring in the retina of the tested eye, and therefrom the machine can determine when the eye properly focuses an image.

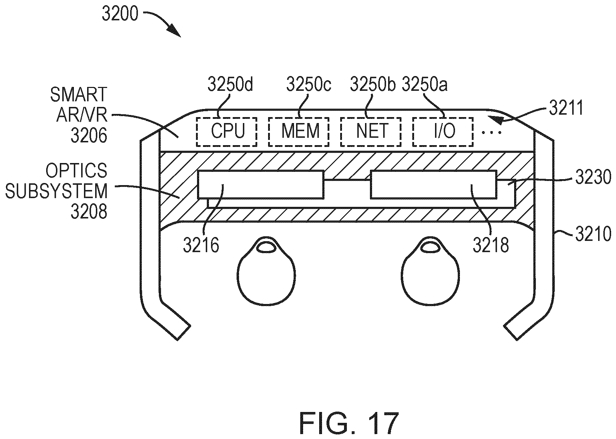

Illustrated in FIG. 17 is a schematic plan view of the architecture of an open view, smart eyewear apparatus 3200 with computer-automated adjustment for visual acuity (at least objective refractive error) embodying principles of the present inventive concepts. The open view, smart eyewear apparatus 3200 includes an eyewear form factor 3210 that houses a computer processor subsystem 3206 and an optics subsystem 3208. Computer processor subsystem 3206 provides the electronics and digital processing components 3250a, b, c, ... n (generally 3250) that implement augmented reality (AR), virtual reality (VR), and mixed reality (MR) presentation of images to the wearer-user at or through translucent viewing surface 3211. Known or common techniques for such augmented reality, virtual reality, and/or mixed reality in smart eyewear technology are employed. Example techniques include: US Pat. No. 11,391,906 to Hudman; US Pat. No. 11,221,479 to Zhao et al; US Pat. No. 10,983,352 to Chan et al; US Pat. No. 10,852,553 to Pedder et al; and WO2022/032198 by Haddock et al.

Optics subsystem 3208 includes tunable optical elements 3216 and 3218, and a working wavefront aberrometer module 3230. Tunable optical element 3216 is part of the lens system for the left eye of the wearer-user, and tunable optical element 3218 is part of the lens system for the right eye of the wearer-user. Tunable optical elements 3216 and 3218 may include fixed power lenses, liquid crystal lenses, liquid membrane lenses, geometric phase lenses, Fresnel lenses, zoom lenses, catadioptric lenses, single-element lenses, multielement lenses, varifocal and reconfigurable meta-lenses (e.g., lenses formed of meta-materials), and/or other lenses, or the like. Fixed power lenses may be formed of glass, polymers, or other material, and power lenses may be lenses that are permanently installed or removably installed (temporarily installed) in the optics subsystem 3208. The working wavefront aberrometer module 3230 is communicatively coupled to the electronics and digital processing components 3250 of the computer processor subsystem 3206.

The eyewear 3210 form factor is of a design that provides an open view to the user. For ease of reference herein, such eyewear is at times called open view smart eyewear 3210 or a pair of smart glasses 3210 meaning eyewear of an open field design.

The digital processing and electronic components 3250 may include a digital processor or CPU 3250d, a network interface 3250b, and supporting memory 3250c, each common in digital processing arts. An I/O (input/output) component 3250a includes sensors, cameras, light source, speakers, microphone, micro display and/or other display subsystem known in digital multi-media and smart eyewear arts. Software supporting the I/O (input/output) electronic elements - component 3250a is stored in memory 3250c and executed by CPU 3250d.

Software supporting other functioning and operations is also stored in memory 3250c and executed by CPU 3250d. The working wavefront aberrometer module 3230 has software instructions executable by CPU 3250d that provide the set (sequence or plurality) of operations implementing automatic adjustment of objective refraction of the wearer-user of the smart eyewear apparatus 3200 as made clearer below. As heretofore unachieved and not contemplated in the art, the working wavefront aberrometer module 3230 provides calibration of smart eyewear 3210 among other advantages.

In embodiments, the working wavefront aberrometer module 3230 generally functions and operates as described below (for hand-held, head worn, stand or other supported portable optical devices) and in PCT International patent application no. PCT/US2022/072186 filed May 6, 2022, published in English on November 10, 2022 as WO 2022/236333, and herein incorporated by reference in its entirety. The working wavefront aberrometer module 3230 determines an autorefractor-like objective measurement of the refractive errors of the user's eyes. Using the objective refractive error measurement, the module 3230 sets initial tunable lens or optical elements 3216, 3218 of the smart eyewear apparatus 3200. Other starting point settings of tunable optical elements 3216, 3218 are suitable. The user's ability to focus an image while wearing the smart eyewear apparatus 3200 is a result of his eyes health (visual acuity) compounded by the correction of the measured refractive error afforded by the tunable lens/optical elements 3216, 3218 of smart eyewear apparatus 3200. Based on user subjective feedback or input, the working wavefront aberrometer module 3230 updates the settings of the tunable lens/optical elements 3216, 3218 to meet the user's (wearer's) optical needs.

Restated, in general, open view smart eyewear apparatus 3200 is a wearable augmented reality device that works like regular non-corrective glasses but includes a digital processor that merges virtual information with physical real-world information to form a resulting presentation to the user. The open view smart eyewear apparatus 3200 and other embodiments of the present invention merge: (a) information regarding user preferences related to eye vision, and (b) information regarding the image as seen by the unassisted eye of this user, to form a subjectively and objectively augmented version of an image presented to the user through the viewing surface 3211 of the open view smart eyewear (smart glasses) 3210. The subjective augmentation is based on the user preferences received as input or feedback to the working wavefront aberrometer module 3230. The objective augmentation is based on the objectively measured refractive error produced by the working wavefront aberrometer module 3230. The resulting augmented image that the smart eyewear apparatus 3200 presents to the wearer-user is representative of (i) the effective visual acuity of the user viewing through the smart eyewear apparatus 3200, and (ii) the net effect of the objective augmentation (refractive error correction through the tunable lens/optical elements 3216, 3218) and subjective augmentation (user preferences communicated through the computer processor subsystem 3206), if any.

The smart eyewear apparatus 3200, via the working wavefront aberrometer module 3230, may continuously and in near real time assess the user's effective ability to focus (refractive power) viewing through the smart eyewear system and automatically adjust the image presented to the user. The automatic adjustments adjust the objective (tunable lens/optical elements 3216, 3218) augmentation and subjective augmentation (if any) of the presented image. The subjective augmentation morphs the subject image toward a target image as a function of user expressed preferences for non-limiting example using digital image enhancement techniques and the like.

Similar to the above-described automatic adjusting of the user's effective visual acuity through the smart eyewear apparatus 3200, the working wavefront aberrometer module 3230 may initially (or at other times) calibrate the smart eyewear 3210 for the specific user. That is to say, some embodiments serve as a smart eyewear calibrator and the like.

Computer automated active adjustment of effective visual acuity of a user wearing the smart eyewear 3210 is heretofore unachieved by the prior art. It is understood that given the disclosure herein it is in the purview of one skilled in the art to use any combination of on-device (smart eyewear apparatus 3200) processing and processing on one or more off-device servers or computers, web or cloud-based servers, computer networks (local area, wide area, or other), and the like where communications with working wavefront aberrometer module 3230 is via network communications interface 3250b. Similarly, such processing may include computations, determinations, and various analyses as described herein or equivalents thereto. Embodiments may employ artificial intelligence or other machine learning techniques to model and thus predict or otherwise enhance the wearer-user preferences as applied to augmenting and presenting images through the smart eyewear apparatus 3200. Other techniques for implementing and affecting the working wavefront aberrometer module 3230 operations and image focusing (augmenting) described herein are suitable.

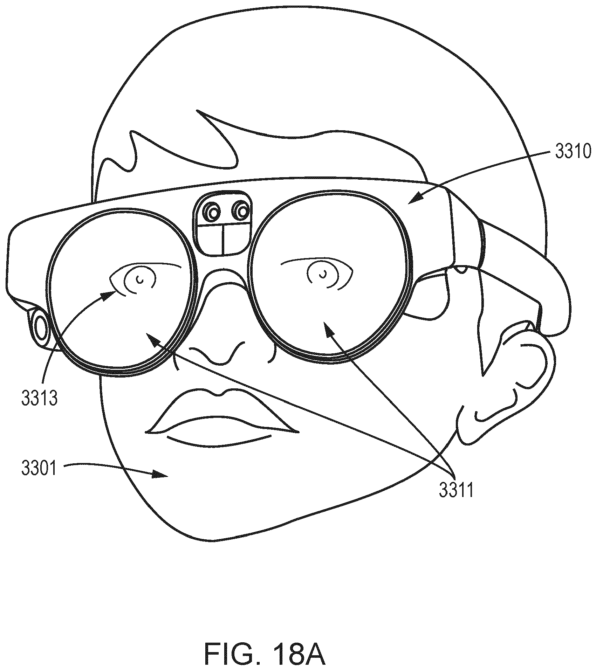

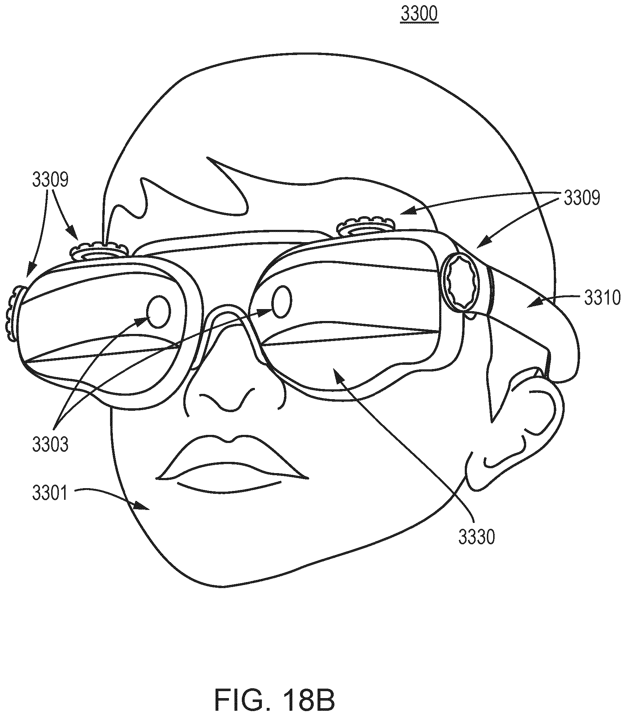

In other embodiments, the working wavefront aberrometer module 3230 may be external to and not built into the open view smart eyewear 3210 form factor. In such embodiments, as an externally located component, the working wavefront aberrometer module 3230 may be removably coupled to the open view smart eyewear. FIGs. 18A and 18B are illustrative.

FIG. 18A shows a user 3301 wearing open view smart eyewear 3310 (for non-limiting example, an augmented reality headset) from a known manufacturer such as Magic Leap Inc. of Plantation FL, USA. The subject smart eyewear 3310 has open view lenses 3311 for the left eye and the right eye (generally, eyes 3313) of the wearer-user 3301. Applicant's tunable optics used to correct the user's refractive errors can be integrated into such open view smart eyewear 3310. As detailed above in FIG. 17 , Applicants tunable optics can be added to the user's eyeball side (or opposite side) of the smart eyewear lenses. In FIG. 18B and described next is an embodiment where Applicant's tunable optics, specifically the working wavefront aberrometer module is operatively coupled and removably secured to the exterior surface of the smart eyewear lenses 3311.

Turning to FIG. 18B, illustrated is the user 3301 wearing open view smart eyewear apparatus or system 3300 embodying aspects of the present inventive concepts. The smart eyewear apparatus or system 3300 is formed of an external working wavefront aberrometer module 3330 temporarily added to the front side of the open view smart eyewear 3310 (specifically to the front surface or exterior facing surface of left eye and right eye lenses 3311 of FIG. 18A). The working wavefront aberrometer module 3330 is removably coupled to the exterior facing lens surfaces of smart eyewear 3310 in a manner that enables modification or calibration of the smart eyewear operations by measuring refractive errors, accommodation amplitude, other vision performance metrics, and/or iris biometrics of the wearer-user 3301.

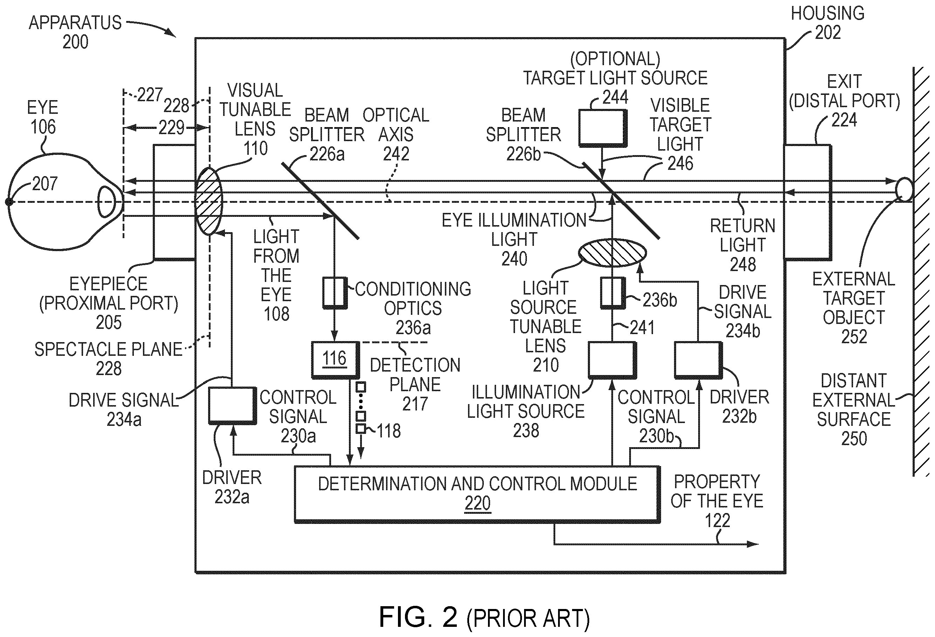

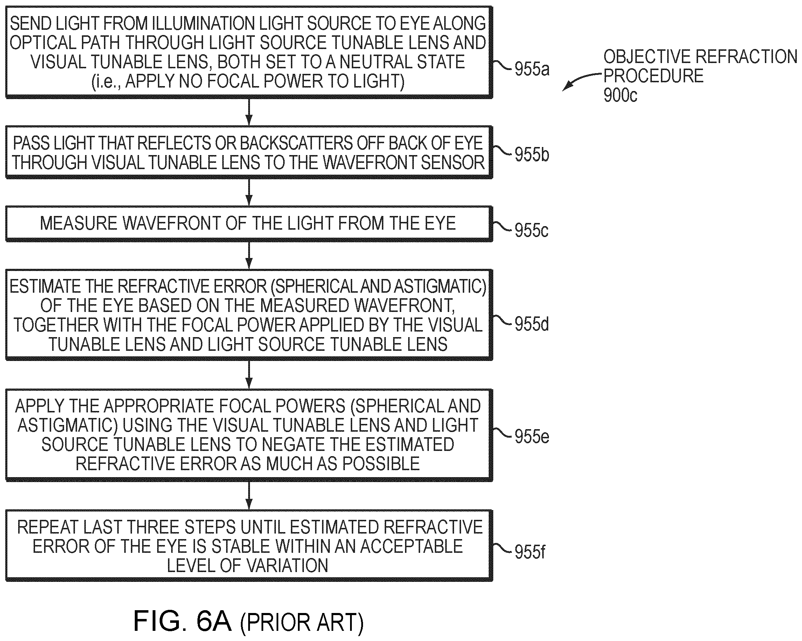

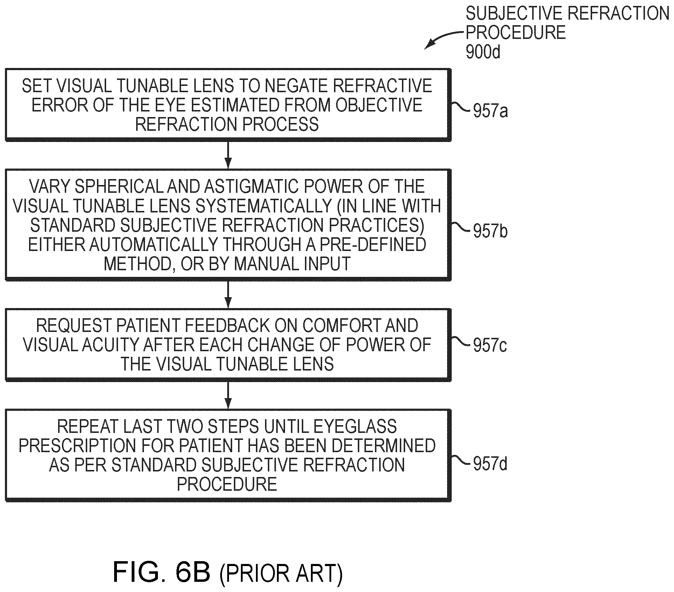

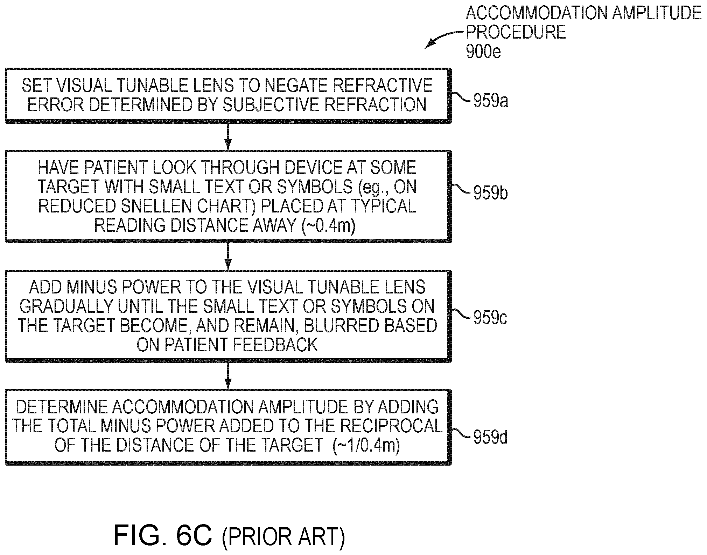

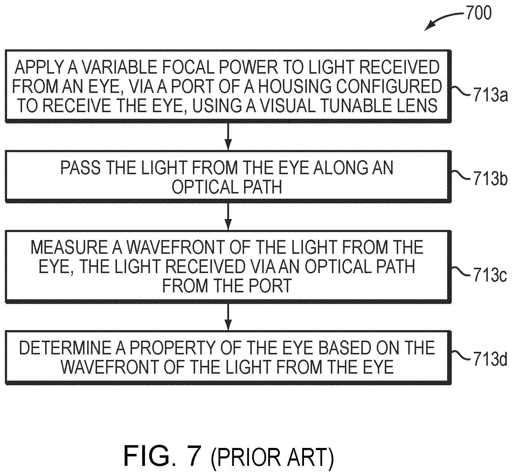

System 3300 performs the calibration by utilizing the refractive power measurements, refractive error measurements, and/or other information from the working wavefront aberrometer module 3330. In particular, the objective refraction procedure described below in FIGs. 7 and 6A is followed by the subjective refraction procedure detailed in FIG 6B , which is followed by the accommodative amplitude procedure detailed in FIG 6C . Other sequence orders of the subjective refraction procedure and the objective refraction procedure are suitable. In turn, as detailed below in FIGs. 2, 4, 10A and 10B, a feedback loop between obtaining objective wavefront measurements and adjusting the system 3300 tunable optics to correct for refraction and to improve user visual performance is employed. In each iteration, the feedback loop updates: (i) the tunable optics contained within the smart eyewear 3310, (ii) the tunable optics in the working wavefront aberrometer module 3330, and/or (iii) the contents displayed to the user in the smart eyewear, for non-limiting example to partially or fully neutralize spherical and/or astigmatic refractive error in the user's eyes 3313. The updates to the system tunable optics and/or display contents dynamically (in near real time and continuously) improve the overall user visual experience and comfort. For instance, the user 3301 may look at a fixed visual target that displays different types of content to simulate different viewing conditions (e.g., perceived distance to the target, contrast, brightness, etc.) , or the target may be moved to different distances and the measurements repeated, or there may be multiple targets to look at, and the like.

The working wavefront aberrometer module 3330 communicates measured objective refraction or corrective vision prescription information to the controller processor of the smart eyewear 3310. In response, the controller processor updates the tunable optics (lens 3311 subsystem) which in turn modify the contents displayed to the user 3301 in a manner that is specific to the user's refractive power/sensed user's ability to focus and that improves the user's visual experience and comfort. Or for smart eyewear 3310 that does not locally employ tunable optics in its lens/optical element subsystem, the controller processor responsively modifies contents displayed to the user in a manner that is specific to the sensed refractive power/sensed ability to focus of the user and that improves the user's visual experience and comfort. In other embodiments, the working wavefront aberrometer module 3330 employs tunable optics responsive to the user's needs. In embodiments, the working wavefront aberrometer module 3330 in near real time and continuously assesses the user's 3301 objective refraction and automatically corrects the user's objective refractive error viewing through the smart eyewear 3310 by so updating and adjusting the tunable optics and/or resulting display of the smart eyewear. In this way, the working wavefront aberrometer module 3330 calibrates the smart eyewear 3310 for use by the specific user 3301.

In embodiments, system 3300 stores the custom calibration parameters and related information in a user record (or profile) in local memory of the smart eyewear 3310, in cloud memory in communication with the smart eyewear 3310, in a mobile app of the smart eyewear 3310, other mobile app of the user 3301, or other suitable computer storage. In embodiments, the user record/profile is associated with the specific user 3301 by iris biometric identification or iris recognition. The smart eyewear 3310 may employ an iris scanning camera (e.g., a pupil camera) as one of its internal components and stores a digital representation of the unique iris pattern of the user 3301 in the user record/profile. Authentication of the user 3301 before each use of the smart eyewear 3310 is then by matching a current scan of the iris of the wearer-user with digital representations stored in user records/profiles. The authentication software of the smart eyewear 3310 associates the identified user with the device (certain pair of smart eyewear 3310) for purposes of security and with device settings (calibration parameters) stored in the identified user's record for purposes of custom configuring/operating the smart eyewear 3310 for current use by this user.

As the working wavefront aberrometer module 3330 can dynamically take measurements while the user looks through the open view smart eyewear system 3300, the dynamic timing and approach of measurement taking improves the measurement capture and resulting data quality. That is, accuracy and specificity of low-order aberration measurements and high-order aberration measurements for use in smart eyewear 3310 is improved over the user 3301 looking through a traditional separate wavefront aberrometer alone. This is because wavefront aberrometry measurements, especially high-order aberrations are sensitive to pupil size which is influenced by the ambient light conditions next to the user's eye. Although refractive error in the user's eyesight may or may not be fully neutralized by sphere, cylinder, and axis tunable optics, high-order aberration information can be used to improve the resulting vision correction and improve visual acuity under certain viewing conditions (such as low light, glare, normal viewing conditions, and the like). Thus, the current and prior art approach of separately measuring a user's refractive errors, while not wearing the smart eyewear, using an autorefractor, photorefractor, phoropter, trial frames, or a wavefront aberrometer, may provide refraction measurements that differ from measurements taken while the user is wearing and looking through the smart eyewear 3310 of interest.

After calibration is completed, the working wavefront aberrometer module 3330 can be decoupled and removed from the smart eyewear 3310. Consequently, the wearer-user 3301 experiences a reduction in overall bulk and weight being worn. In particular, working wavefront aberrometer module 3330 may include pupil imaging optics, keratometry optics, iris biometric identification optics, tunable optics, a digital processor, computer memory, and/or a battery/power unit needed to run the working wavefront aberrometer module components, related software, and the interface to smart eyewear 3310. Decoupling and removing the working wavefront aberrometer module 3330 from smart eyewear 3310 after calibration reduces the bulk and weight that the working wavefront aberrometer module 3330 added to smart eyewear 3310.

Continuing with FIG. 18B, the working wavefront aberrometer module 3330 includes ports 3303. The ports 3303 serve or function as an extension and continuation of the open view design of the smart eyewear 3310. Adjustment actuators 3309 or similar adjustment mechanisms provide alignment of a line of sight for the eye of the user 3301 through smart eyewear lenses 3311, the working wavefront aberrometer module 3330, and ports 3303. The adjustment actuators 3309 enable independent or separate manual alignment of each optical channel (one for each eye) formed by the lenses 3311, working wavefront aberrometer module 3330, and ports 3303.

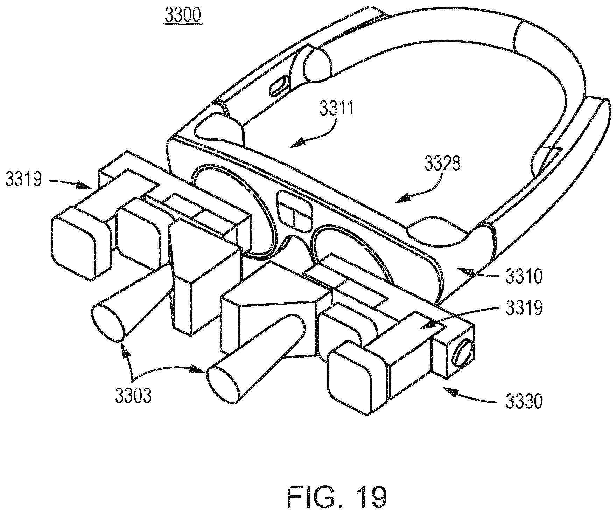

FIG. 19 shows a schematic view of the smart eyewear system 3300 of FIG. 18B with the external working wavefront aberrometer module 3330 placed in front of and removably coupled to the open view pair of smart eyewear 3310. The working wavefront aberrometer module 3330 enables custom modification or calibration of the smart eyewear 3310 by measuring refractive errors, accommodation amplitude, other vision performance metrics, and/or iris biometrics of the wear-user as discussed above. In embodiments, the working wavefront aberrometer module 3330 includes pupil imaging optics, keratometry optics, iris biometric identification optics, and/or tunable optics. Reference is made to FIGs. 2, 8, 9, and 11 below for more detailed examples of the layout of these optical components. In turn, working wavefront aberrometer module 3330 performs wavefront aberrometry, pupil imaging, keratometry, iris biometric imaging, and/or user-specific tuning of its optical elements (if any) as described herein. Ports 3303 of the working wavefront aberrometer module 3330 provide an open view for the wearer-user. The wearer-user looks at displayed content and at the real world through smart eyewear lenses 3311 (together with ports 3303 at times). There may be a native pupil camera 3328 built into the smart eyewear 3310.

For simplicity, mechanical alignment mechanisms 3319 are schematically shown and adjust the vertical and horizontal (X axis and Y axis) position and the pantoscopic tilt and angle of the removably coupled working wavefront aberrometer module 3330 on each lens 3311 (right eye side and left eye side) of smart eyewear 3310. The mechanical alignment mechanisms 3319 are operated through adjustment actuators 3309 described above in FIG. 18B. The mechanical alignment mechanisms 3319 align the ports 3303 of the working wavefront aberrometer module 3330 with the eyes of the user looking through the smart eyewear lenses 3311 at a distant target.

After user-specific or custom calibration of the smart eyewear 3310, the working wavefront aberrometer module 3330 is decoupled and removed from smart eyewear 3310. During subsequent use of the smart eyewear 3310, information from the native eye tracking or pupil camera 3328 may be used to perform gaze tracking of the user and assess where the user is looking. There will be a convergence (angle of the two eyes relative to each other) change when the user is looking at a distant object and not requiring accommodative power (for non-limiting example, more than 10 feet away from their eyes) versus when the user is looking at an object that is close to their face (for non-limiting example, less than 1.5 feet away from their eyes). The detected convergence change enables the smart eyewear 3310 to utilize the information from the user-specific calibration that is pre-loaded into the smart eyewear memory to update the smart eyewear tunable optics or to update the content displayed to the user through the smart eyewear 3310 in a manner accommodating the user's vision needs.

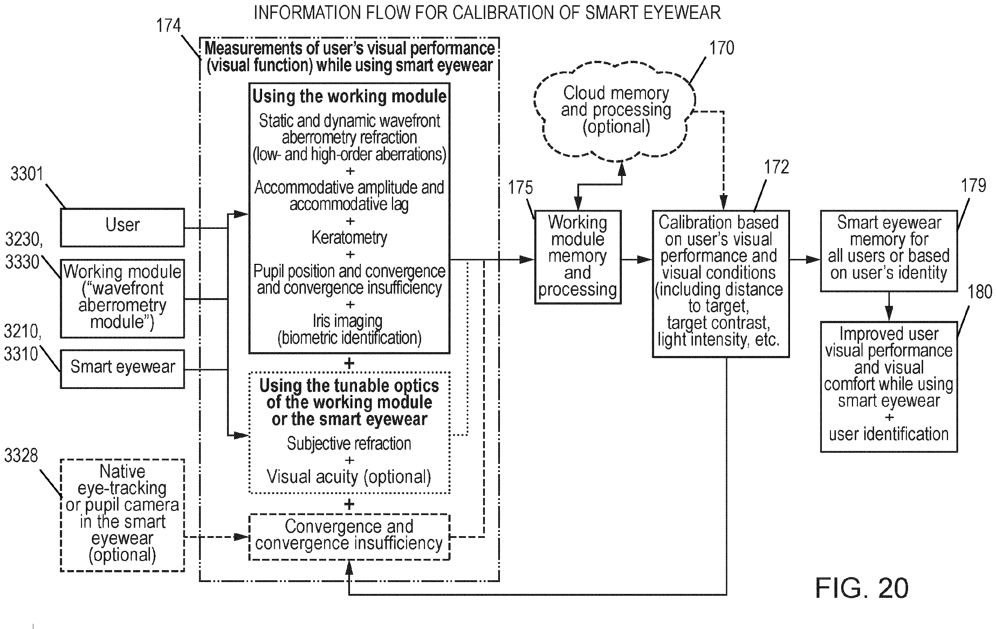

As detailed above in FIGs. 17-19 , some embodiments of the working wavefront aberrometer module 3230, 3330 are removably coupled to smart eyewear 3310 and some embodiments 3230 may be built in (an internal component) to a pair of open view smart eyewear 3210. In either system 3200, 3300, working wavefront aberrometer module 3230, 3330 provides custom calibration 172 specific to the wearer-user as described next in FIGs. 20-22. FIG. 20 illustrates the general flow of measurements and information (including pupil tracking and detected convergence) 174 to calibrate the smart eyewear 3210, 3310 to a user's vision performance (visual function) while looking through the smart eyewear and working wavefront aberrometer module 3230, 3330 under a single or multiple conditions of interest. Say for non-limiting example, a technician at a point of sale, service center, and the like uses or applies the working wavefront aberrometer module 3230, 3330 as a calibration tool configuring the tunable lens 3311 or optic elements 3216, 3218 of the smart eyewear 3310, 3210 specifically for the user. The working wavefront aberrometer module takes user-specific measurements and information 174 and responsively determines calibration parameters at processing step 175. The resulting custom calibration parameters 172 and related information are stored in local memory of the smart eyewear 3210, 3310, cloud memory 170 in communication with the smart eyewear, or the like as illustrated in the middle of FIG. 20.

The smart eyewear 3210, 3310 may employ an iris scanning camera as one of its internal components and stores a digital representation of the unique iris pattern of the user 3301 in the user record/profile 179. Authentication of the user 3301 before each use of the smart eyewear 3210, 3310 is then by matching a current scan of the iris of the wearer-user with digital representations stored in user records/profiles 179. The authentication software 180 of the smart eyewear 3210, 3310 associates the identified user with the device (certain pair of smart eyewear 3210, 3310) for purposes of security and with device settings (calibration parameters) stored in the identified user's record for purposes of custom configuring and operating the smart eyewear 3210, 3310 for current use by this user. The right-hand side of FIG. 20 is illustrative.

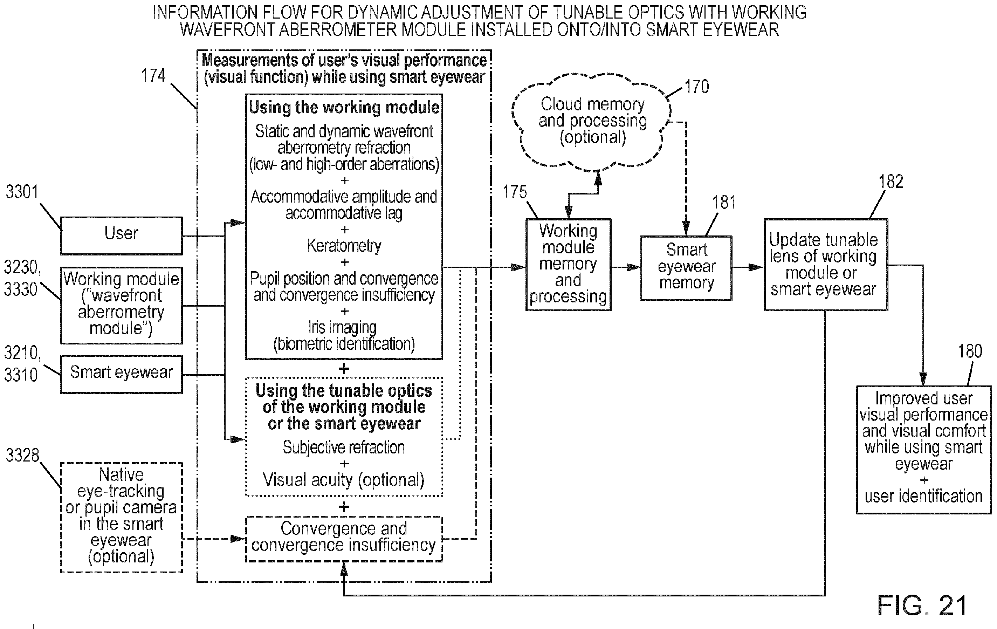

In another embodiment, the working wavefront aberrometer module 3330 is configured for removable attachment to the smart eyewear (pair of open view smart glasses) 3310 by the user and serves as an optional mobile app accessory of sorts. In this mode, the user temporarily applies the working wavefront aberrometer module 3330 to personalize (calibrate and configure through the mobile app) the tunable optics of the smart eyewear 3310 before use. The working wavefront aberrometer module 3330 takes user-specific measurements and information 174 and responsively determines calibration parameters at processing step 175. The resulting custom calibration parameters and related information are stored 181 in local memory of the smart eyewear 3310, mobile app file storage, cloud memory 170 in communication with the mobile app or smart eyewear, or the like as illustrated in FIG. 21. The stored custom calibration parameters are used to update 182 the tunable optic elements (lens) 3311 of the smart eyewear 3310. Authentication of the user 3301 before each use of the smart eyewear 3310 is by matching a current scan of the iris of the wearer-user with digital representations stored in user records/profiles. The authentication software 180 of the smart eyewear 3310 associates the identified user with the device (certain pair of smart eyewear 3310) for purposes of security and with device settings (calibration parameters) stored in the identified user's record for purposes of custom configuring and operating the smart eyewear 3310 for current use by this user.

In yet another embodiment, the working wavefront aberrometer module 3230 is built in and coupled with the internal components of the smart eyewear 3210 such as in system 3200 of FIG. 17 detailed above. FIG. 21 shows the general flow of measurements and information 174 to adjust the tunable optics 3216, 3218 with the built-in working wavefront aberrometer module 3230 of the smart eyewear 3210 in a manner that improves the wearer-user's vision performance and comfort while using system/apparatus 3200. The system 3200 adjusts (such as at 182) the tunable optics 3216, 3218 based on the user-specific calibration under a single or multiple visual conditions. In this mode, the embodiment system/apparatus 3200, i.e., the combined working wavefront aberrometer module 3230 and smart eyewear 3210 with open view form factor, dynamically and continuously applies Applicant's wavefront aberrometry techniques (e.g., obtaining measurements 174/ processing/ generating calibration parameters at step 175) during user use of the system/apparatus, and responsively adjusts 182 the tunable optics and/or display of the smart eyewear 3210 while the user is viewing objects at different depths or light conditions (contrast levels, etc.). In this way, the embodiment system/apparatus 3200 custom tunes the smart eyewear 3210 for multiple depths. The resulting custom calibration parameters and information are stored 181 in local memory 3250c of the smart eyewear system 3200 or optionally on cloud memory 170 in communication with the smart eyewear 3210. Authentication of the user before each use of the smart eyewear system 3200 may be by matching a current scan of the iris of the wearer-user with digital representations stored in user records/profiles. The authentication software 180 of the smart eyewear system 3200 associates the identified user with the device (certain pair of smart eyewear 3210) for purposes of security and with device settings (calibration parameters) stored in the identified user's record for purposes of custom configuring and operating the smart eyewear system 3200 for use by the specific user. FIG. 21 is illustrative.

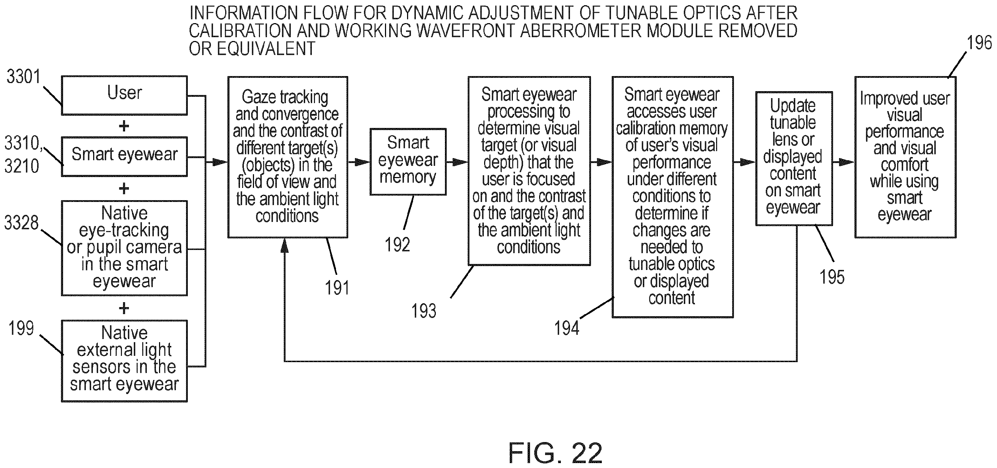

After custom calibration (especially after decoupling and removal of the external working wavefront aberrometer module 3330 in smart eyewear system 3300), FIG. 22 illustrates the general flow of measurements and information to adjust the tunable optics within smart eyewear 3310, 3210 to improve a wearer-user's visual function (performance in vision) and comfort while the user is looking through the smart eyewear. The smart eyewear system 3200, 3300 adjusts 195 the tunable optics 3216, 3218, 3311based on the user-specific calibration under a single or multiple visual conditions. External light sensors 199 of the smart eyewear 3210, 3310 detect lighting conditions. In embodiments, the pupil camera 3328 tracks user gaze and detects a convergence change at 191 while the user is viewing through the smart eyewear 3210, 3310. In response, the processor of the custom calibrated smart eyewear 3210, 3310 determines visual depth that the user is focused on and determines lighting conditions at 193 using sensor data and pupil camera data stored192 in smart eyewear memory. Using determined visual depth and lighting conditions information as input, the processor accesses 194 the smart eyewear memory and custom calibration information including the user's vision performance under different conditions. In turn, the processor determines 194 changes needed to update the tunable optics or displayed content. The processor performs the updates 195 to the tunable optics or displayed content which has the effect of improving 196 the user's experience (vision performance and comfort) while viewing through the smart eyewear 3210, 3310. The smart eyewear system 3200, 3300 in near real time and continuously tracks the eye gaze of the wearer-user and automatically corrects for the user's objective refractive error (vision needs specific to the user) viewing through the smart eyewear system by so adjusting and updating the tunable optics and/or resulting display to the user.

### Wavefront Aberrometer (from US Pat. No. 11,096,576)

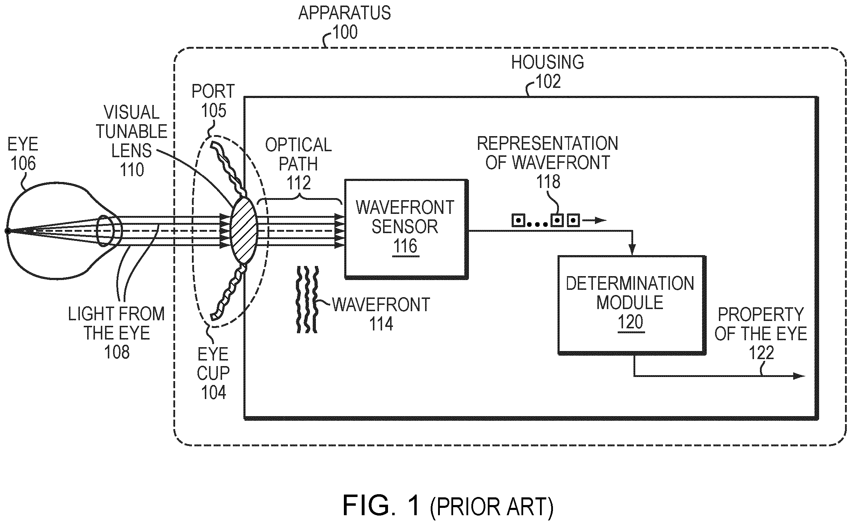

FIGs. 1- 7 and 10A-10B (prior art) are drawings from US Pat. No. 11,096,576, granted on August 24, 2021, which is hereby incorporated by reference in its entirety. US Pat. No. 11,096,576 teaches various features that may be advantageously used in embodiments of the present application, and to which reference may advantageously be made in understanding the scope and details of the present embodiments. A paraphrase, restatement, or otherwise repeat of the pertinent parts of US Pat. No. 11,096,576 and corresponding description of FIGs. 1- 7 and 10A-10B follow in the next subsection. Where the term "patient" or "patient-user" is used relative to features and devices of embodiments, it is understood that the end-user of the smart eyewear or wearer of the same is intended or synonymous in the context of this disclosure.

Refractive eye examinations by an optometrist or ophthalmologist typically involve using a phoropter to determine which of many fixed lens settings produces the best eyesight, subjectively, for a given patient. Clinical phoropters are usually binocular (enabling both of a patient's eyes to view through separate sets of lenses) and open-view (enabling a patient to view, through the phoropter lenses, a distant target pattern). Typically, the patient is asked to focus on a target pattern situated a distance of about 20 feet from the patient's eye. The open-view design also performs the important function of encouraging the patient's eyes to remain unaccommodated (relaxed and as optimized as possible for long-distance viewing) during the measurement. The unaccommodated state is an important clinical prerequisite for accurate measurements for refractive correction of distance vision. Thus, using typical clinical phoropters, a prescription for refractive correction can be obtained that both (i) corrects for important types of optical aberrations of the eyes and (ii) takes into account a patient's subjective feedback about which correction is preferable.

Wavefront aberrometers, in contrast to clinical phoropters, determine a refractive correction for a patient objectively, without input from the patient, based on sensing a wavefront of near infrared (near-IR) directed into the eye and reflected or scattered from the retina of the eye. Wavefront aberrometry in an eye clinic can provide information about aberrations of the eye of higher order than just sphere and cylinder and is considered a valuable tool in determining refractive correction.

Handheld devices have been developed more recently to perform wavefront aberrometry. A goal of these devices is to enable refractive examination of people in remote areas without access to standard clinics or eye care professionals, as well as to decrease cost of examination and limit the number of expensive instruments required, as well as to streamline the refractive examination in high-resource settings. Handheld wavefront aberrometer devices have some limitations, in that they do not take into account subjective feedback from an eye patient. Further, high-quality clinical phoropters may not be available to supplement handheld device measurements, due to the cost, size, weight, and mobility limitations of standard phoropters.

Moreover, an important clinical requisite for an accurate measurement is that the patient's eye should be relaxed while the measurement is made. Since existing handheld devices do not allow a patient to view through them and focus on a distant object to cause eye relaxation, there are a number of other techniques that have been used to induce relaxation using existing non-open view devices. For example, cycloplegic drops can be placed in the eye to paralyze accommodative control. While effective, these drugs may often have side effects that can be undesirable for the patient and can require 15 minutes or more for their effects to occur. Another approach is to place lenses in front of the patient's eyes to simulate myopia (i.e. shortsightedness), referred to as "fogging" the patient, so that the patient's eye(s) are coerced into relaxing their accommodation so as to bring a fixation target into focus. While fogging can be effective for many patients, some others may not respond well to this technique. Furthermore, these techniques, even when effective, still do not produce exactly the same results as actually allowing a patient to view a distant target through an open view lens, as is done with standard clinical phoropters.

Including a set of physical lenses, similar to those of a phoropter, with a handheld device has been attempted. However, this increases cost, weight, and system complexity, and this solution also has feasibility challenges because switching between lenses requires at least some mechanical motion or disturbance of a portable handheld device. Thus, this approach would be less mechanically robust and prone to breaking or mis-alignment. Alternative optical approaches such as adaptive optics (e.g., deformable mirrors, spatial light modulators) are prohibitively expensive for application in low-cost diagnostics.

Presented or subject wavefront aberrometer apparatus examples of U.S. Patent no. 11,096,576 can include one or more tunable lenses integrated into a handheld wavefront aberrometer to act effectively as an on-board phoropter. Wavefront aberrometer measurements can be used as feedback to the tunable lens, in closed-loop fashion, to automatically and quickly adjust a tunable lens through which the patient views to optimize vision objectively. Because the tunable lens can iteratively correct measured wavefront errors until the measured wavefront is nominally parallel, objective wavefront evaluations can be made with greater accuracy. As will be understood by those familiar with Hartmann-Shack wavefront sensors, for example, when the wavefront is nominally parallel, a spot pattern produced by the sensor has spots nominally uniformly spaced. In this state, uniformity of the spot pattern (and, hence, wavefront errors) can be more exactly evaluated than if the spot pattern is very distorted.

After objective autorefraction, feedback from a patient can be used to further adjust the tunable lens in accordance with subjective patient preference to improve the proposed correction. An apparatus can communicate with a patient through a variety of methods to obtain the subjective feedback, even automatically or semi-automatically. This feedback can be obtained and implemented in embodiment devices with or without the assistance of a technician or other eye professional. Subject wavefront aberrometer embodiments can be designed to be self-usable by a single user (i.e., eye patient) without assistance from a clinician (e.g., an ophthalmologist, optometrist, clinical assistant, field technician, or any other person working to assist an eye patient to obtain a corrective prescription).

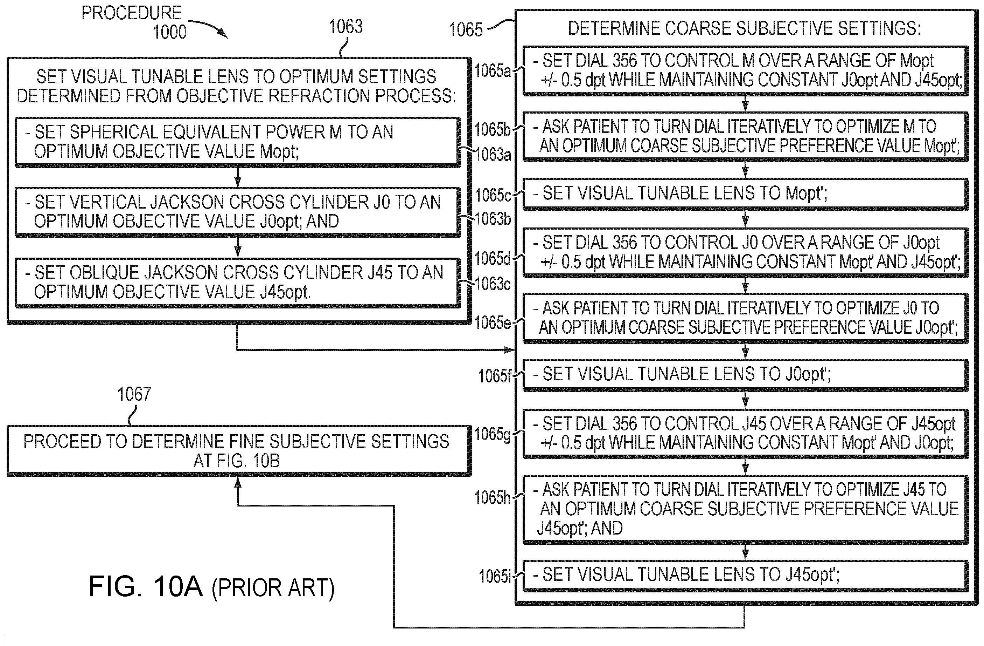

Self-usable wavefront aberrometer embodiments are made possible in part because the eye can be aligned with the optics of the device via an external or internal fixation target, or visual or audio cues from the device, or both. Self-usable embodiments can be further enabled by automated or semiautomated operation of embodiment apparatuses and interactive instruction to a patient and saving settings made by a patient. FIGs. 10A-10B, as described hereinafter, provide one example of such an interactive procedure consistent with some embodiment apparatuses. However, subject wavefront aberrometers disclosed herein may also be modified and used advantageously with clinician assistance to obtain results that are similarly unachievable by other handheld units and further unachievable by using mutually separate phoroptry and aberrometry instruments.

Thus, integrated autorefraction and phoropter functions allow the phoropter core (the tunable lens) to be automatically updated based on the autorefraction wavefront data. Furthermore, when the tunable lens is appropriately situated in or on the device incorporating the wavefront sensor, the tunable lens can serve as an eyeglass simulation to allow a patient to see through a lens at the appropriate location for an eyeglass with the final, proposed correction prescription implemented. Moreover, certain subject wavefront aberrometers can be used to take advantage of the tunable lens to measure accommodation and presbyopia, as well as to perform lensometer functions by measuring optical parameters for a set of eyeglasses already owned by the patient or to be offered to the patient, for example. Furthermore, the lenses to be measured using subject wavefront aberrometer devices may be lens blanks as well. An optician may want to locate the optical center position and confirm the power of the lenses before cutting the lens blanks to fit an eyeglass frame, for example.

Subject wavefront aberrometer devices are preferably "open view," meaning that the patient can see through the device to a distant target to relax any accommodation of the eye. A subject wavefront aberrometer apparatus can be designed to measure the aberrations of the user's eye in an unaccommodated state (i.e. the eye is relaxed and focused at infinity). A viewing target may be located at effective optical infinity, about 20 feet from the patient's eye. Viewing targets can include a standard eye chart, a spot of light produced by a target light source on the device, or another object in the surrounding environment. Such open view designs can reduce or eliminate the need for cycloplegic drops, and fogging may also be rendered unnecessary or optional.

By using a relatively low-cost, electronically tunable lens system, expensive approaches such as adaptive optics can be avoided. Subject wavefront aberrometer devices can also be more mechanically robust and easier to handle and transport than systems having a set of physical lenses included in a phoropter, for example. A standard phoropter-style or trial frame lens system requires lens switching, which can result in fluctuations of the patient's eye and mechanical disturbances to the wavefront sensor apparatus.

It is noteworthy that available tunable lenses may have lower optical quality than fixed lenses typically used in high-quality optical systems for eye care. For this reason, optical engineers and eye care professionals would not generally be inclined to consider using a tunable lens in a system designed for high-quality eye examination, whether a phoropter or a wavefront aberrometer. However, the inventors have recognized that, where a device is designed for a tunable lens to work in combination with a wavefront sensor, the quality of wavefront aberrometry can be maintained and even enhanced due to iterative wavefront measurements in the presence of automatic, closed-loop, wavefront error cancellation by the tunable lens. Subject wavefront aberrometers can provide wavefront measurements that are captured and processed continuously, such as at video rates, for example. Furthermore, a wavefront sensor apparatus can be calibrated with respect to any wavefront error caused by a tunable lens, thus enabling measurement of even high-order wavefront errors of a patient's eye with high accuracy. Furthermore, as noted hereinabove, a tunable lens also enables fast automated or semi-automated phoropter, lensometer, and accommodation measurement functions on the same device that is used for wavefront aberrometry, even with a portable, handheld device, and even when testing is self-administered by the patient.

Subject wavefront aberrometers can provide a complete refraction system that enables refractive measurements to be performed anywhere by a minimally-trained technician or even by the subject patient himself or herself. This has significant global health and industrial utility.

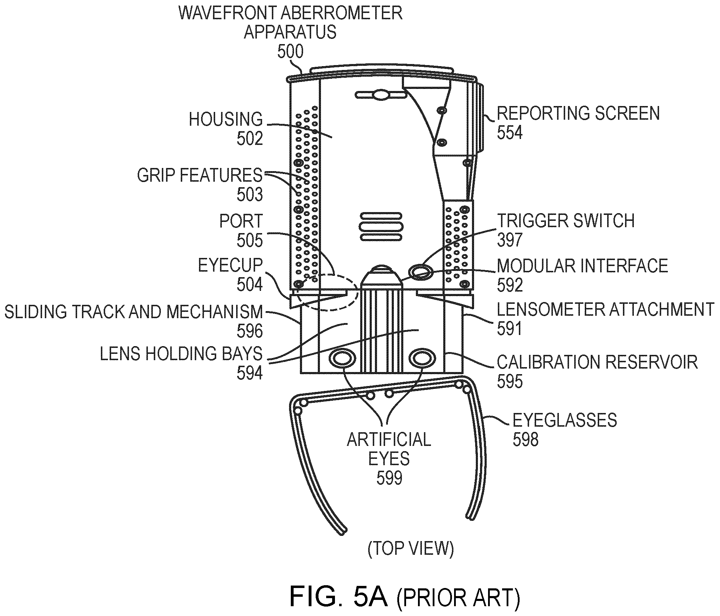

FIG. 1 is a schematic block diagram illustrating subject wavefront aberrometer apparatus 100 for determining a property of an eye 106. The apparatus 100 includes a housing 102 having a port 105 configured to receive the eye 106 and to receive light 108 from the eye. The port 105 is "configured to receive" the eye 106 in the sense that the eye 106 can be placed near enough to, or in contact with, one or more portions of the port such that the light 108 from the eye can be received through the port 105. Thus, while the eye 106 is not required to be in contact with the port 105, in various embodiments, the eye 106 is an eye of a person whose forehead and cheek are placed against an eyecup 104 for registration and mechanical fixation with respect to the port 105. As a further example, another embodiment device having an eyecup configured to come into contact with a person's forehead and cheek is described hereinafter in connection with FIGs. 5A-5C. Other subject wavefront aberrometers may or may not have an eyecup. Some embodiments defining a binocular configuration may include two ports, also referred to herein as "first" and "second" ports, that include similar configurations and provide for similar functionalities for first and second respective eyes of a patient, as described in connection with FIGs. 5A-5C, for example.

The apparatus 100 in FIG. 1 also includes a visual tunable lens 110 mounted to the housing as part of the port 105. The visual tunable lens 110 is designated "visual" because it is possible for the eye 106 to see through the visual tunable lens 110. The visual tunable lens 110 is also configured to focus or defocus light received from the eye 106 to be passed via an optical path 112 to a wavefront sensor 116, which measures a wavefront of the light 108 from the eye. The "visual" tunable lens 110 is also closer to the eye 106, when the apparatus 100 is in use than an optional "light source tunable lens" that will be described in connection with FIG. 2, and which can be a similar tunable lens at a different location in the apparatus. In various embodiments, the visual tunable lens 110 mounted in the apparatus such that it is relatively close to the eye 106 when the apparatus is brought into proximity with the eye for examination. A smaller relative separation between the tunable lens and the eye can result in the tunable being smaller and less expensive than would otherwise be needed.

The visual tunable lens 110 has a focal length f and an optical power P = 1/f that are variable. In some embodiments, the visual tunable lens 110 is configured to apply a variable spherical power (focus/defocus) to the light 108 from the eye. In other embodiments, the visual tunable lens 110 can also apply astigmatic power (cylinder) and also vary axis of the cylindrical (astigmatic) power applied to the light. In some embodiments, the visual tunable lens can be configured to apply variable spherical and astigmatic optical powers, as well as apply axis orientation for the astigmatic power, mutually independently. In some embodiments, the visual tunable lens 110 is further configured to apply a spherical equivalent power, vertical Jackson cross cylinder, and oblique Jackson cross cylinder mutually independently.

It should be understood that any "tunable lens," as used herein, can include a plurality of individual tunable lenses arranged (optically stacked) in series, along the same optical axis, for example. Individual tunable lenses can be stacked in series (along the same optical axis) in order to increase the range of lens powers that can be simulated by the system. Stacking of tunable lenses may also improve the dynamic range or reduce the overall aberrations of the system. For example, a visual tunable lens may include a first individual tunable lens with a wide range of coarse tunability for a given optical correction such as sphere, as well as a second individual tunable lens with a narrow range of fine tunability for the given optical correction. Further, the optional mutual independence of spherical and astigmatic powers with variable axis may be achieved by applying the powers and axis using respective individual tunable lenses. This same method of using individual tunable lenses can be used to apply spherical equivalent power, vertical Jackson cross cylinder, and oblique Jackson cross cylinder mutually independently.

In some subject wavefront aberrometers, the visual tunable lens 110 can be at least one of a liquid-filled lens, an electro-wetting lens, an Alvarez lens, spatial light modulator, deformable mirror, a lens with power that varies spatially (e.g., a progressive lens), a multi-lens system that changes lens distances to tune optical power (e.g., optical trombone, Badal system), or a tunable Fresnel lens. In some subject wavefront aberrometers, the visual tunable lens can include a two-element object configured to apply the variable focal power as a function of lateral or rotational displacement of the two elements with respect to each other. For example, an Alvarez lens pair can include two such optical elements configured to be laterally displaced with respect to each other, in a direction perpendicular to an optical axis of the elements, to apply the variable focal power. Another embodiment wavefront aberrometer includes a lens that is tunable by virtue of being asymmetrical having different focal powers along different points on the lens. Such an asymmetrical lens can be displaced along a plane perpendicular to an optical axis of the system in order to vary the focal power of the lens. Asymmetrical lenses of this type have been termed "hybrid Fresnel lenses" and have been used in virtual reality headsets, for example.

Example tunable lenses that can be used in embodiments described herein can include, for example, the Optotune^{®} EL-10-30 series of liquid-filled tunable lenses. This series has focal lengths and corresponding optical powers that can be tuned within milliseconds, providing fast response for iterative wavefront measurements performed in a closed loop fashion, as further described hereinafter. One model of the Optotune^{®} EL-10-30 can be tuned between +8.3 and +20 diopters (dpt) of optical power, corresponding to +120 to +50 mm in focal length, for example. Furthermore, the Optotune^{®} EL-10-30 series is available with near-infrared (NIR) optimization, which is useful for detecting NIR light received from the eye, as is preferably done in some embodiments. Tunable lenses can also cover negative power ranges to be used with myopic patients. The Optotune^{®} EL-10-30-C-NIR-LD-MV, for example, can be tuned between -1.5 and +3.5 dpt. Another example tunable lens that can be used includes the Varioptic Visayan^{®} 80S0 electro-wetting tunable lens, which can apply variable focus (-12 to +12) and astigmatism (-6 to +0 dpt) powers.

In FIG. 1, the visual tunable lens 110 is also configured to pass the light 108 from the eye along the optical path 112 toward the wavefront sensor 116. The wavefront sensor 116 is configured to receive the light from the eye and to measure a wavefront 114 of the light 108 from the eye. The wavefront sensor 116 can be, for example, a Hartmann-Shack wavefront sensor comprising an array of lenslets having the same focal length and configured to focus light received, at various points in a cross-section of a beam of light, onto a photon sensor, which can be a CCD or CMOS array, for example. As is known and understood in the art of wavefront sensing, such a wavefront sensor produces a pattern of spots, from which a wavefront of the light being measured can be determined with high precision.

The wavefront sensor 116 provides a representation 118 of the wavefront of the light 108 to a determination module 120. The representation 118 of the wavefront can include, for example, an image in the form of pixel values for a sensor array of the wavefront sensor 116. However, in other subject wavefront aberrometers, the wavefront sensor 116 can be configured to provide the representation 118 in other forms, such as a compressed image or a series of spot separations or spot center positions on the sensor array, for example.

The determination module 120 is configured to determine a property 122 of the eye 106 based on the measured wavefront from the sensor 116. The property 122 can include an optical property such as one or more values for aberrations of the eye, an eyeglass or contact lens prescription for the eye, objectively or subjectively determined correction parameters, accommodation amplitude or presbyoptic prescription, lensometer data for eyeglasses worn or intended to be worn by a patient, or other related data. Moreover, in some embodiments, the determination module 120 can be configured to output other data, such as a spot pattern produced by the wavefront sensor 116. Such spot patterns can be used advantageously in some embodiments to provide live images for alignment of the eye and other purposes, as described further hereinafter.

In some subject wavefront aberrometers, the housing 102 is configured to be gripped by at least one hand of the person having the eye 106 to support a full weight of the apparatus during use. An example of such a configuration is included in FIGs. 5A-5C, for example. These embodiments can enable a person having the eye to use the apparatus 100 portably, even in the absence of a doctor, operator, or other assistance to obtain eye data such as a prescription for eyeglasses.

In some subject wavefront aberrometers, the port 105 can include an optical window in the housing 102 or can be an opening in a modular attachment to the housing. In some embodiments, the eyecup 104, the visual tunable lens 110, and the port 105 can be physically separate. In some embodiments, port 105 can be described as a "proximal" port, and an additional "distal" port can also be provided in the housing, such that the device is "open view," enabling the eye 106 to see all the way through the apparatus 100 to an object or feature external to the apparatus 100. The apparatus 100 is monocular, in the sense that it is configured to receive one eye. However, in other subject wavefront aberrometers, an apparatus can be binocular, as described hereinafter in connection with FIGs. 5A-5C, for example. In some binocular configurations, a second visual tunable lens can be configured to be mounted to the housing and to apply a variable focal power to light from the second eye. The second visual tunable lens can perform functions similar to those of the first visual tunable lens 110, or separate functions, as will be described further hereinafter.

In some subject wavefront aberrometers, an apparatus can include a visual tunable lens configured to be adjusted iteratively to optimize the wavefront 114. For example, the visual tunable lens 110 can be adjusted to make the wavefront 114 as close as possible to a plane wavefront, such that aberrations produced by the eye 106 can be minimized, and the visual tunable lens 110 can simulate an eyeglass lens worn by a person having the eye 106.

In some subject wavefront aberrometers, the eye 106 is a living eye of a person. However, in other embodiments, the eye 106 is an artificial eye that can be used for calibration purposes, for example, or for determining the prescription of a pair of eyeglasses in accordance with lensometer functions, as further described hereinafter in connection with FIGs. 5A-5C.

FIG. 2 is a schematic block diagram illustrating a subject wavefront aberrometer apparatus 200 that is configured to be open view and also include other optional features. Open view embodiments have the advantage that the eye 106 can view target indicia external to, and spaced away from, a housing 202 of the apparatus 200 through a visual channel between two sides of the apparatus, as further described hereinafter. Provided an external target object 252 at a distant external surface 250, or other target indicia, are spaced away from the eye 106 at effective infinity (greater than or equal to 20 feet from the eye), the eye 106 can remain substantially unaccommodated and relaxed, such that refractive measurements performed by the apparatus 200 can be improved. It has been shown that wavefront aberrometry with a closed view configuration induces more instrument myopia (0.3 dpt) compared to an open view system (e.g., A. Cervino et al., Journal of Refractive Surgery, 2006).

The apparatus 200 is configured to have the visual tunable lens 110 mounted within close proximity to an eyepiece 205 serving as a proximal port configured to receive the light 108 from the eye 106 through the housing 202. The eyepiece 205 is detachable from the housing 202, such that it is modular and can allow the housing 202 to receive other modular attachments. Example modular attachments can include a lensometer attachment, as described hereinafter in connection with FIGs. 5A-5C, a calibration attachment, or other eyepieces having different focal ranges.

As is known, different eyes can have widely varying optical aberrations and require widely varying prescriptions. A given visual tunable lens having a given tunability range, such as the Varioptic Visayan^{®} 80S0 tunable lens described hereinabove, which has an adjustment range from -12 to +12 dpt, will be able to simulate eyeglass corrections for patients having a given range of needed correction. Thus, in some subject wavefront aberrometers, the eyepiece 205 with the visual tunable lens 110 covering one range of corrections can be modularly replaced with another eyepiece having a different tunable lens covering a different range of corrections to address patients having a correspondingly different range of correction.

Alternatively, in some subject wavefront aberrometers, the eyepiece 205 is configured to accommodate additional lenses and optics for various purposes. For example, the eyepiece 205 can be configured to accommodate a fixed lens, also attached to the housing, to apply a fixed focal power to the light 108 from the eye to shift a range of refractive correction measurement of the apparatus 200. Furthermore, a variety of fixed lenses having various fixed focal powers can be alternately received by the eyepiece 205, or by another portion of the housing 202, or inside the apparatus 200, for example, to address different persons with different refractive corrections. Furthermore, the eyepiece 205 can also be configured to accommodate a fogging lens or optic configured to fog the view of the eye through the apparatus 200. Fogging has the advantage that it is a non-cycloplegic (does not require cycloplegia) approach and also avoids the need for an open view system. Fogging can also be modified according to a given patient's type of refractive error (myopia or hyperopia).

Still further, the eyepiece 205 can be configured to accommodate a visual tunable lens that comprises a series of individual tunable lenses as described hereinabove in relation to FIG. 1. Using a series of individual tunable lenses instead of a single visual tunable lens, for example, may increase the range of lens powers that can be simulated by the system. A series of individual tunable lenses may also improve the dynamic range or reduce the overall optical aberrations of the system. The individual tunable lenses may be arranged (stacked) optically in series with each other, all centered on a common optical axis, for example. The individual tunable lenses may be used to cover separate larger and smaller optical correction ranges, for example. Further, individual tunable lenses in such an arrangement (stack) may be configured to address, separately, different respective optical corrections. A series of individual tunable lenses can separately address spherical power, astigmatic power (cylinder), axis of the cylindrical (astigmatic) power applied to the light, and even aberrations of higher optical order mutually independently, for example. In some embodiments, the series of individual tunable lenses can be configured to apply a spherical equivalent power, vertical Jackson cross cylinder, oblique Jackson cross cylinder, and higher-order corrections mutually independently.

As is understood in the science of refractive care, corrective lenses of eyeglasses are typically situated about 14 mm from the surface of the cornea of a patient's eye. In some wavefront aberrometer embodiments, in order to best simulate refractive correction of eyeglasses, the visual tunable lens 110 is configured such that a plane 228 of the lens 110 is configured to be a distance 229 of about 14 mm from a front surface 227 of the cornea. Thus, the plane 228 at which the visual tunable lens 110 is situated, in this case, corresponds to the spectacle plane for the eye 106 when the proximal port has received the eye.

While a refractive measurement is being performed, the eye 106 can see light 248 from the external target object 252 located on the surface 250 at effective infinity. This open view design is facilitated by two beam splitters 226a and 226b that perform various functions within the apparatus and are also largely transparent in the visible spectrum perceived by the eye 106.

The beam splitter 226a is configured to reflect NIR light 108 received from the eye 106 toward the wavefront sensor 116. The optical path between the beam splitter 226a and the wavefront sensor 116 also includes various conditioning optics 236a. The conditioning optics 236a can include, for example, a beam aperture/iris, a narrowband optical filter configured to pass only NIR light of a given wavelength, an attenuation filter, etc. The conditioning optics 236a can also optionally include cross-polarizers disposed in the optical path and configured to minimize unwanted light at the wavefront sensor 116. In the case of a beam aperture, light from the eye illumination light source can be restricted by the aperture, and example aperture sizes may range between about 50 µm and about 500 µm.

The wavefront sensor 116 provides the wavefront representation 118 to a determination and control module 220, which is configured to determine the property 122 of the eye. The determination and control module 220 performs functions similar to those of determination module 120 in FIG. 1, but the module 220 also includes control functions. In particular, the control module 220 outputs a control signal 230a to a lens driver 232a, which outputs a drive signal 234a to the visual tunable lens 110 to set the lens 110 to the appropriate focal power. With appropriate logic in the determination and control module 220, this forms a closed-loop system (circuit), wherein the wavefront representation 118 can be continuously monitored, and wherein the control module 220 can provide appropriate control signals 230a to update the setting of the visual tunable lens 110 continuously. This process can be iterative to minimize wavefront errors of the eye 106 using the visual tunable lens 110. In this manner, the variable focal power of the visual tunable lens may be adjusted iteratively in response to successive wavefront measurements in order to minimize a wavefront error of the light from the eye. Various iterative processes are further described hereinafter in connection with FIGs. 10A-10B, for example.

The apparatus 200 also includes an illumination light source 238 that is configured to output NIR light (eye illumination light 240) toward the eye. In other embodiments, the eye illumination light and light received from the eye may be visible or infrared. The illumination light 240 is reflected by the beam splitter 226b, passes through the beam splitter 226a, and exits the proximal port 205 through the visual tunable lens 110 to enter the eye 106. The light 240 is intended to form a focused spot 207 at the retina of the eye 106. A portion of the eye illumination light 240 is reflected and scattered by the eye 106 and is received as light 108 from the eye to be detected at the wavefront sensor 116.

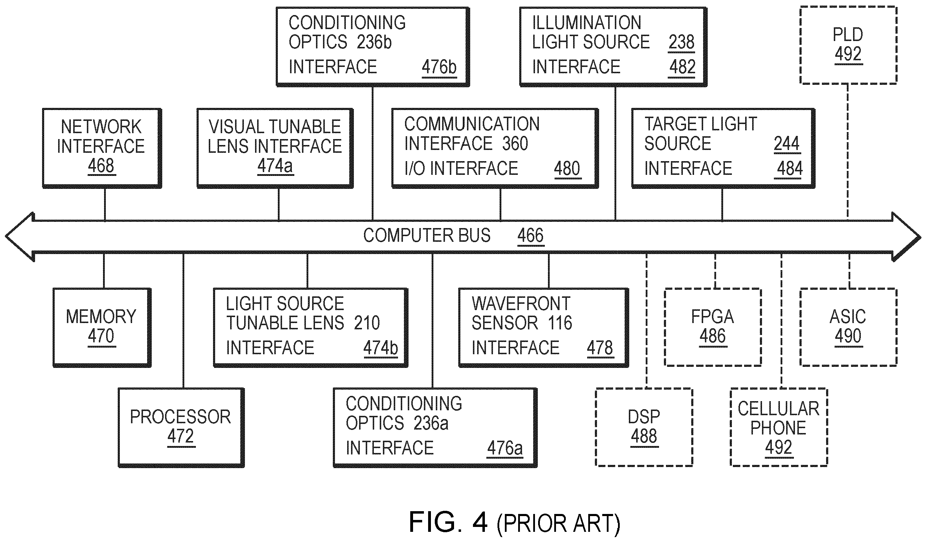

When the eye illumination light 240 passes through the visual tunable lens 110, its convergence or divergence is affected by the setting of the tunable lens 110. In order to maintain a focused spot 207 at the retina, the apparatus 200 includes a light source tunable lens 200 that applies variable focal power to the eye illumination light 240 to maintain the focused spot 207 at the retina. Thus, when the determination and control module 220 adjusts the focal power of the visual tunable lens 110, the light source tunable lens 210 can be adjusted to a corresponding value that affects only the eye illumination light 240 and maintains the focused spot 207. As will be understood by those skilled in the art of optics, the corresponding settings between the visual tunable lens and the light source tunable lens 210 can be pre-calibrated such that an appropriate setting for the lens 210 can be known for every setting of the tunable lens 110. In order to make these corresponding adjustments, the determination and control module 220 can store calibration data or receive the calibration data from another source, such as memory illustrated in FIG. 4, to make the appropriate corresponding settings.

In cases in which the visual tunable lens 110 can correct over the refractive error range needed for a given patient, corresponding adjustments to the light source tunable lens 210 may not be required. However, the light source tunable lens 210 can be used to extend the range of measurement for a given visual tunable lens 110 by reducing spot size of illumination light focused onto the retina of the eye, particularly in case the eye has a refractive error greater in magnitude than the maximum refractive error that can be corrected with the visual tunable lens 110. Furthermore, the light source tunable lens 110 can be used to expedite analysis of a patient's eye and determination of a corresponding prescription by sweeping the range before, during, or after tuning the visual tunable lens. For example, if a particular visual tunable lens cannot be tuned as fast as desired for a given set of refractive measurements, then the optical power of the light source tunable lens may be adjusted, in parallel with the optical power of the visual tunable lens, to achieve a particular combined power setting more quickly. Moreover, the light source tunable lens 210 can be used to reduce speckle, as described further hereinafter.

In order to control the light source tunable lens 210 in FIG. 2, the determination and control module 220 outputs a control signal 230b to a lens driver 232b. The driver 232b outputs a drive signal 234b to the light source tunable lens 210 to make the appropriate setting. Preferably, where the visual tunable lens 110 controls sphere, cylinder, and axis independently, the light source tunable lens 210 includes similar, independent adjustments such that the eye illumination light can remain focused on the retina for all visual tunable lens settings.

The optical path between the illumination light source 238 and the beam splitter 226b also includes conditioning optics 236b. The optics 236b can include some functions similar to those of the conditioning optics 236a. For example, the optics 236b can include a narrowband filter configured to pass only light of wavelengths corresponding to the illumination light source 238. The optics 236b can also include an iris (aperture) configured to adjust diameter of the eye illumination light 240 or a diaphragm to define the illumination light and to align the light 240 with the beam splitter 226b. The illumination light source 238 can be a light emitting diode (LED), but it can also be a diode laser or other collimated, coherent (or semi-coherent, such as a superluminescent diode) light source, for example.

As will be understood by those skilled in the art of optics, a coherent illumination light source 238, such as a laser, can produce some degree of speckle pattern at the eye 106 and at the wavefront sensor 116, depending on the degree of coherence of the light source 238. Random speckle patterns with high contrast may, therefore, be present in a spot diagram produced using the wavefront sensor. These speckle patterns can interfere with the ability of the wavefront sensor 116 to distinguish sensitively between laser speckle and the spot pattern that defines the wavefront of the light 108. Speckle contrast can reduce the accuracy of localizing each spot in a detected spot diagram, which, in, turn can reduce the accuracy of a wavefront that is reconstructed using the detected spot diagram.

One advantage of embodiments is that the determination and control module 220 can be configured to dither (i.e., rapidly apply variable focal power or adjust another refractive setting of) either the visual tunable lens 110, the light source tunable lens 210, or both tunable lenses slightly while spot diagrams are being acquired by the wavefront sensor. In the case of the light source tunable lens being dithered, variable focal power is applied to the light 241 from the light source 210. This dithering can randomize the speckle pattern produced by the eye illumination light 240 at the eye 106, or, equivalently, randomize a speckle pattern produced by the light 108 from the eye at the wavefront sensor 116. This dithering can introduce small variations into the wavefront of the light to randomize the speckle pattern generated at the eye by an eye illumination light source and received at the wavefront sensor.

Such dithering can reduce or eliminate the effects of laser speckle pattern that would otherwise diminish measurement sensitivity of the wavefront sensor 116. If the magnitude of the dithering is sufficiently large, the speckle pattern will be randomized over the course of an acquisition. If the speckle pattern is sufficiently randomized over the course of a single exposure, an averaged-out speckle pattern will be captured. This implies that the spots in the spot diagram can be more accurately localized due to the reduced speckle contrast. Furthermore, a dithering magnitude that is sufficiently large to randomize the speckle pattern can also be small enough to have no appreciable impact on the size of the focal spot 207 or the accuracy of the wavefront measurements. An example spherical dithering magnitude includes, for example, +/- 0.01 dpt. However, other example spherical dithering magnitudes are much greater, such as in a range of 0.25-0.5 dpt, for example. Other tunable lens parameters, such as cylinder power, axis, higher order parameters, or parameters such as spherical equivalent power in other known basis sets, for example, may be dithered as an alternative to, or in addition to, dithering sphere. Thus, the ability to eliminate or reduce laser speckle noise is yet another advantage of tunable lenses used in embodiment apparatus and methods.

The apparatus 200 also includes an optional target light source 244 mounted to the housing 202. FIG. 2 illustrates the target light source mounted inside the housing 202, but other embodiments can include outside mounting. The target light source 244 is configured to output visible target light 246, which is reflected by the beam splitter 226b and output from the apparatus 200 through a distal port 224 in the housing. Together, the proximal and distal ports form a visual channel parallel to the optical axis 242 through which the eye 106 can see the external target 252. The visible target light 246 creates a spot or other indicia on the distant external surface 250 external to and spaced away from the housing 202. The spot or other indicia can be viewed by the eye 106 to cause the eye to be unaccommodated, with the distant external surface 250 at effective infinity from the eye. The visible target light 246 is reflected or scattered from the surface 250, and a portion returns to the eye 106 as return light 248 through the apparatus 200. However, in other wavefront aberrometer embodiments, the target light source 244 is not used. Instead, the return light 248 viewed by the eye 106 is ambient light scattered or reflected from the external target object 252 and through the apparatus 200.

In the schematic block diagram illustrated in FIG. 2, the light 108 from the eye, visible target light 246, return light 248, and eye illumination light 240 are shown as being offset from the optical axis 242 of the eye. This depiction is for convenience in illustration only, and all of these light beams can be mutually coincident, collinear, and centered on the optical axis 242.

However, in some wavefront aberrometer embodiments, the eye illumination light 240 exiting the port 205, and the light 108 from the eye entering the port and received by the tunable lens 110, are non-collinear. This non-collinear orientation can reduce or eliminate eye illumination light 240 that is back-reflected from the surface of the cornea of the eye from being received at the wavefront sensor. This can be very helpful in reducing noise and increasing signal-to-noise ratio for wavefront signals detected by the wavefront sensor.

In conformity with the principle of making the light entering the eye non-collinear with the light exiting the eye, various adjustments can be made to the optical configuration in FIG. 2. For example, a detection plane 217 of the wavefront sensor 116 can be non-perpendicular to an illumination axis 241 of the illumination light source 238. The wavefront sensor 116 can be slightly non-parallel with the optical axis 242 of the eye. In other words, the detection plane 217 of the wavefront sensor can be non-parallel with an illumination axis of the eye illumination light 240 within an optical path between the beam splitter 226b and the eye 106, and the detection plane 217 can be non-perpendicular with an axis of illumination of the eye illumination light 240 within an optical path between the eye illumination light source 238 and the beamsplitter 226b.

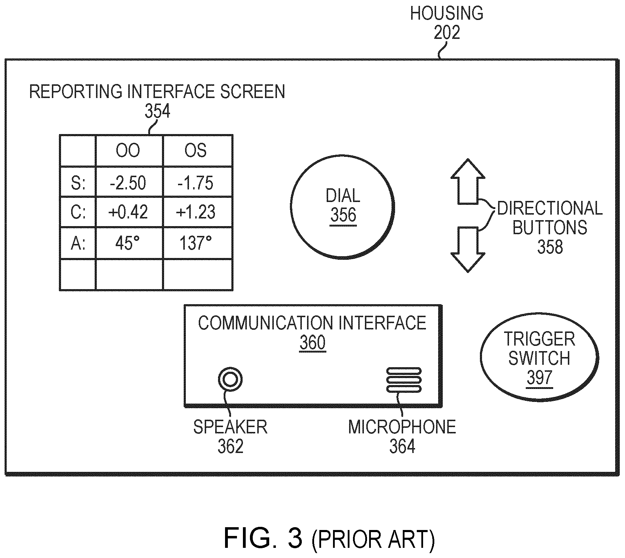

FIG. 3 is a schematic diagram illustrating various optional input and output features of subject wavefront aberrometer devices, such as those illustrated in FIGs. 1 and 2. In particular, the housing 202 of the apparatus 200 illustrated in FIG. 2 can include a reporting interface screen 354, a dial 356, a communication interface 360, directional buttons 358, and a trigger switch 397. The dial, directional buttons, and trigger switch are examples of manual controls that can be configured to be adjustable by an eye patient, or by a clinician, to adjust the variable focal power of the visual tunable lens in accordance with a subjective refractive preference of the eye patient. In other embodiments, these inputs and outputs are provided by peripheral devices in operational communication with the apparatus 200. Examples of peripheral devices, can include a cellular phone, as illustrated in FIG. 4, or a separate, handheld, wired or wirelessly connected controller that a clinician, patient or other user can use to specify inputs or receive outputs, for example.

The reporting interface 354 can be an LCD screen, for example, on the housing 202 that can be read by a user to obtain a prescription for eyeglasses, as illustrated, or another property of the eye 106. The reporting interface screen 354 provides sphere (S), cylinder (C), and axis (A) measurements for right (OD) and left (OS) eyes after measurements are completed. Various other information can also be presented to a user or operator using the reporting interface screen 354, such as information about higher order aberrations, Zernike polynomial parameters measured for the right and left eyes, a contact lens prescription, alignment information, and other information. As another example, the reporting interface screen 354 can show a live image produced by the wavefront sensor 116 in FIG. 2 to assist with calibration of the apparatus or for eye alignment purposes for initial setup, for example. Further alternative information that can be provided by the reporting interface screen 354 includes static images produced by the wavefront sensor 116, other information representative of the wavefront detected, calibration instructions, operating instructions, etc. Furthermore, in some embodiments, the reporting interface screen 354 is a touchscreen enabling a user to input information, such as selecting a measurement to be performed. Actual placement of the features shown in FIG. 3 onto a device housing or peripheral module may vary in various embodiments. An example placement of the trigger switch 397 is illustrated in FIGS. 5A-5C.

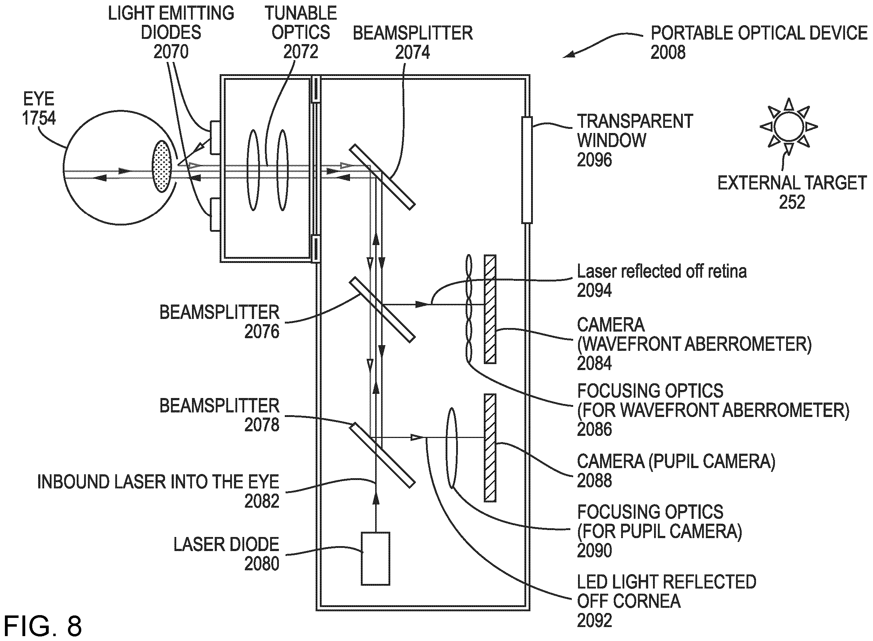

The communication interface 360 includes a speaker 362 configured to provide audible instructions to a user, such as instructions for how to align the eye to an input port of the housing for best measurement accuracy. In some embodiments, the speaker 362 provides step-by-step instructions to the user before and during a measurement of the eye. The interface 360 also includes a microphone 364 that can be used to receive inputs from the user, such as a refractive preference of the user. This feature is particularly useful when the apparatus 200 operates in phoropter mode, as described in FIGs. 8A-8B, of U.S. Patent no. 11,096,576 for example. Thus, the speaker 362 can provide certain instructions such as "tell me which lens setting is best, one or two." The apparatus 200 illustrated in FIG. 2 can then set the visual tunable lens to two different settings, one subsequent to the other, and the speaker 362 can indicate which setting is 1 in which setting is 2. A user can then speak, through the microphone 364, "one" or "two" to indicate which setting of the visual tunable lens 110, simulating an eyeglass correction, is preferable to the user, who is the person whose eye 106 is being measured.

As an alternative to the verbal communication just described for specifying subjective preferences, the directional buttons 358 can be pressed by a user to specify which visual tunable lens 110 setting is preferable. For example, the wavefront sensor 116 can be used to determine an objective refractive correction for the user. The visual tunable lens 110 can then be set to simulate a corrective lens applied to the eye 106. The user can then be given the opportunity to specify various changes to refractive settings of the visual tunable lens 110, using the directional buttons 358, in accordance with a subjective preference. This range of adjustment can be a fine adjustment over a relatively small range, such as a spherical correction adjustment range of +/- 0.25-0.50 dpt. Once the user has specified spherical correction to the subjective preference, the buttons 358 can then be used to optimize cylinder and axis in turn according to subjective preferences, in a similar fashion. After the visual tunable lens 110 is set to all the preferred settings for sphere, cylinder, and axis, the process can be repeated iteratively for greater precision or to evaluate repeatability of subjective preference settings.

The dial 356 can be used as an alternative to the directional buttons 358. For example, the user can turn the dial 356 to adjust the spherical correction over the limited range of +/- 0.25 dpt or +/-0.50 dpt, for example. The dial 356 can be preferable to the directional buttons 358 since rotational motion of the dial 356 can be smoother and cause less disturbance to the housing 202 than pressing buttons. The dial 356 may also be easier to use for other reasons, such as the user's ability to turn the dial 356 quickly or slowly, in accordance with the user's preference and the degree of adjustment required.

The trigger switch 397 provides another means of input by the user to the apparatus. In particular, as further described hereinafter in connection with FIG. 5A, for example, the trigger switch 397 can be pressed by the user when the user is ready for a measurement to occur, and then the user can release the trigger switch 397 once a simulated refractive correction provided by the visual tunable lens 110 operating in closed-loop fashion with the wavefront sensor is completely satisfactory. An example location for the trigger switch 397 is shown on the embodiment device illustrated in FIG. 5A.

FIG. 4 is a computer interconnect diagram illustrating various components of the determination and control module 220 in FIG. 2 and its connections to various components, including some internal components shown in FIG. 2 and other optional components shown in FIG. 3, as well as some other optional components that are not illustrated in FIGs. 2-3. In the apparatus embodiment illustrated in FIG. 2, the determination and control module 220 performs all necessary computing and control functions for the apparatus 200. It should be noted that in other wavefront aberrometer embodiments, these functions can be distributed between a determination and control module and other processors or controllers, as will be understood by those skilled in electrical and computer engineering.

The determination and control module 220 includes a computer bus 466 used as an interconnect for various components. The module 220 includes memory 470 and a processor 472 that are used to store data and program instructions and perform necessary processing functions, processing functions can include determining the property of the eye, such as optical properties including a refractive correction prescription to be applied to the eye, based on the measured wavefront, the tunable lens setting, and any objective preference information obtained. The representations 118 of the wavefront entering the module 220 in FIG. 2 can be stored in the memory 470 for analysis by the processor 472. The module 220 also includes a network interface 468 coupled to the computer bus 466 for communicating with outside computers or networks if desirable. The network interface 468 can be used to report refractive results to an external computer or network for eyeglass ordering purposes, for example, or allow the functioning of the apparatus 200 to be monitored by an external or even remote computer, for example.

The processor 472 is coupled to a visual tunable lens interface 474a that controls the driver 232a illustrated in FIG. 2. Thus, through the visual tunable lens interface 474a, the processor 472 can control the settings for the visual tunable lens 110. In a similar fashion, the processor 472 is coupled to a light source tunable lens interface 474b for control of the light source tunable lens 210 illustrated in FIG. 2. It should be understood that, where either the visual tunable lens 110 or the light source tunable lens 210 includes a series of individual tunable lenses, as described hereinabove in relation to FIG. 1, either interface 474a or 474b may correspondingly include a series of individual interfaces for mutually independent control of the respective, individual tunable lenses.

The module 220 also includes interfaces 476a and 476b to control the conditioning optics 236a and 236b, respectively. The interfaces 476a-b are particularly useful in cases in which the conditioning optics are adjustable. For example, the conditioning optics 236a-b can include such features as variable attenuation and adjustable diaphragms and irises for beam conditioning.

The module 220 also includes a wavefront sensor interface 478 for receiving data from the wavefront sensor 116 in FIG. 2. A communication interface 480 in the module 220 allows the module 220 to communicate data to and from the communication interface 360 illustrated in FIG. 3. While not shown in FIG. 4, other interfaces can be provided in the determination and control module 220 for sending data to, and receiving data from, the reporting interface screen 354, dial 356, directional buttons 358, and trigger switch 397, which are illustrated in FIG. 3. Interfaces 482 and 484 are also included in the module 220 for controlling the illumination light source 238 and the target light source 244, respectively, which are illustrated in FIG. 2. For example, these light sources may be turned off when not in use, and their intensity may also be adjustable in certain embodiments.

The network interface 468 can include a wired or wireless interface, such as a universal serial bus (USB) interface, a wired Ethernet interface, a bluetooth communication module, a wireless infrared (IR) interface, a wireless local area network (WLAN) interface, or a wireless cellular data interface. Through such example interfaces, the processor 472 can communicate with an external or remote device that is outfitted with a similar communication interface. Such an interface can be used to print eye measurement results, store results on a thumb drive or other storage medium, send measurement results to a personal computer, cellular phone, smart phone, or cloud-based server, send prescription orders for eyeglass or contact lens prescriptions via any of these or other known means, communicate in other ways, or provide other output data. In one example, objective refraction results, subjective refraction results, lensometry results, accommodation measurements, another eye property, machine learning results, or a combination thereof, as determined by any one or more of the procedures illustrated in FIGs. 7, 6A-6C, and 10A-10B, may be communicated directly or indirectly to a desired location or device with the network interface 468 being configured appropriately. In smart eyewear embodiments, additional output information includes: keratometry results, keratoconus measurements, pupil imaging, iris biometric identification information. The biometric identification can be used for identification in general, or, to identify the user and pre-load their calibration (custom calibration) to improve the response of the smart eyewear to their visual performance and thus improve their visual experience and comfort.

One or more of the interfaces illustrated in FIG. 4 can be replaced or have its functions augmented by a suitably programmed device, such as an optional field-programmable gate array (FPGA) 486 or a digital signal processor (DSP) 488. Furthermore, an application-specific integrated circuit (ASIC) 490 or programmable logic device (PLD) 492 can also be used.

As also illustrated in FIG. 4, the module 220 can include an interface used to communicate with a cellular phone 492. In some embodiments, the cellular phone can be configured to be attached to the housing 202 or can be otherwise programmed to perform some of the functions described in connection with FIG. 2 for the determination and control module 220. Furthermore, in some embodiments, the cellular phone 492 is used to display a representation of the wavefront of the light from the eye. Such a representation can be used for alignment of the eye to the apparatus 200 or for other subjective or objective analytical purposes, for example. In some embodiments, the cellular phone 492 can be used to perform the functions of the reporting interface screen 354 shown in FIG. 3, as well as other input or output functions of the dial 356, communication interface 360, directional buttons 358, or trigger switch 397. Furthermore, in some embodiments, the cellular phone can be used as a Hartmann-Shack wavefront sensor. For example, a standard multi-pixel sensor array on the cellular phone that is used to acquire photographs can be adapted to perform the functions of the light sensor array of the Hartmann-Shack wavefront sensor, and a separate lenslet array can be used to focus the light 108 received from the eye onto the sensor array. In some embodiments, the cellular phone includes two multi-pixel sensor arrays that are used as respective Hartmann-Shack wavefront sensors for respective eyes of a patient. Further, a first one of the two sensor arrays may be used as a wavefront sensor, while a second one of the two sensor arrays may be used to perform one or more of pupil measurements, keratometry, iris imaging, or other known ophthalmic imaging functions.

FIG. 5A is a top-view illustration of a subject, binocular, wavefront aberrometer apparatus 500. The apparatus 500 is particularly configured to enable not only wavefront aberrometer measurements using the visual tunable lens 110 as illustrated in FIG. 2, but also to enable lensometer measurement functions. The apparatus 500 includes a housing 502, which includes grip features 503 configured to be gripped by at least one hand of a person having the eye 106 to support a full weight of the apparatus 500 during use.

Connected to the housing 502 is an eyecup 504 configured to provide mechanical registration of the apparatus 500 against a forehead and cheek of a person (user, patient) having the eye 106. A port 505 in the housing is configured to receive the eye 106 and to receive light from the eye, as described in connection with FIG. 2. The trigger switch 397 is mounted to the housing 502 as illustrated in FIG. 5A. The switch 397 performs the functions as described in connection with FIG. 3. In particular, when a user is ready for the apparatus 500 to perform a measurement, the user presses the trigger switch 397. After the trigger switch is pressed, successive wavefront measurements are obtained by the wavefront sensor 116 illustrated in FIG. 2, and the determination and control module 220 adjusts the visual tunable lens 110 to simulate eyeglass correction.

Each time the visual tunable lens 110 is adjusted, the light source tunable lens 210 can be adjusted by a compensating amount to cause the eye illumination light 240 to form a focused spot 207 at the retina of the eye 106. These adjustments can be performed iteratively, until the user is satisfied with the simulated refractive correction. Once the user is satisfied, the user can again press the trigger switch 397 to indicate that the correction is satisfactory. In other subject wavefront aberrometers, the user or a technician or other person assisting can press and hold a trigger switch while iterative adjustments are performed, and release of the trigger switch can indicate that a user is satisfied with the correction.

The apparatus 500 also includes reporting screen 554 that is configured to display a lens prescription intended for the patient (user). In various embodiments, the reporting screen 554 can be configured to display a contact lens prescription, a wavefront spot pattern for alignment or other purposes, or other information described in connection with the reporting interface screen 354 illustrated in FIG. 3, for example.

FIG. 5A also shows a lensometer attachment 591, modularly attached to the apparatus 500 via a modular interface 592, for performing lensometer measurements for eyeglasses 598. The housing 502 is thus configured to receive a lensometer attachment 591 that is configured to receive and support a corrective lens intended to be worn by a person. The lensometer attachment 591 can also be configured to support a lens blank that is intended to be manufactured into a corrective lens; in this way, the lensometer attachment 591 is useful for both lensometer measurements in a clinical setting and for analysis of lenses and lens blanks during a lens manufacturing process. The wavefront sensor can measure the wavefront of the light received through the corrective lens or lens blank. A determination module, such as module 120 in FIG. 1 or module 220 in FIG. 2, can be configured to determine a refractive property of the corrective lens or lens blank based on a lens wavefront of light received through the corrective lens or lens blank.

In FIG. 5A, the lensometer attachment 591 includes lens holding bays 594 for placement of the pair of eyeglasses 598, with each lens in its own isolated bay. A calibration reservoir 595 including artificial eyes (model eyes) 599 is also included in the attachment 591 for aligning two optical components of known optical wavefront properties to two respective optical channels in the apparatus 500. The calibration reservoir 595 may also be referred to as calibration holder or calibration bay.

The attachment 591 in FIG. 5A also includes a sliding track and mechanism 596 between the modular interface 592 and the calibration reservoir 595 to clamp the optical components of the eyeglasses 598 in a manner to minimize movement and stabilize the eyeglasses for lensometer measurements. The sliding track and mechanism 596 can be used to set a distance between the two channels of the binocular apparatus 500. When the apparatus 500 is used to determine a refractive correction for someone's eye, the sliding track and mechanism 596 can be used to adjust the binocular apparatus 500 to match the interpupillary distance (i.e., the distance between the eyes of the user). When the apparatus 500 is used for lensometry on a pair of eyeglasses, then the sliding track and mechanism 596 can be used to match the binocular apparatus 500 to a distance between respective optical centers of the two lenses of the eyeglasses. The trigger switch 397 also causes the apparatus 500 to trigger a lensometer measurement through the initiation of a software calibration sequence.

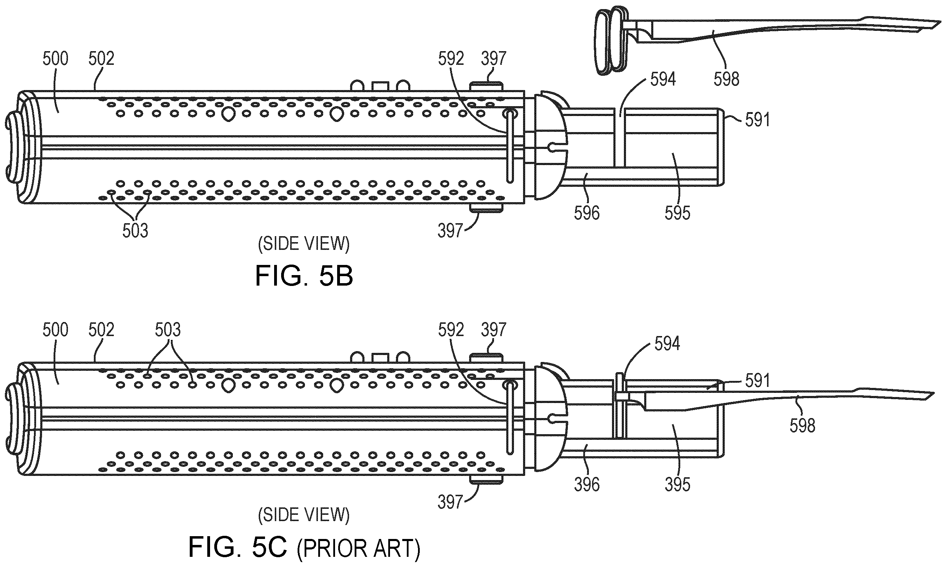

The artificial eyes 599 are shown included in the calibration reservoir 595 for calibration purposes. The artificial eyes 599 can act as known aberrations so that aberrations due to the eyeglasses can be determined. The lensometer attachment 591 is further described below in connection with FIGs. 5B-5C. In particular, there can be reservoirs in which to hold the artificial eyes and slots in which eyeglass lenses can be placed.

The tunable lens 110, which is used in the apparatus 500 for eye measurement purposes as further described herein, can be optionally used or removed from the apparatus for lensometer purposes. Where the tunable lens 110 is used, it can be held at a fixed optical power so as to shift the measuring range of the apparatus 500 in case the eyeglass lens being measured fall outside the base range of the apparatus.

FIGs. 5B-5C are side-view illustrations of the apparatus 500 illustrated in FIG. 5A. In particular, FIG. 5B shows the eyeglasses 598 outside of the lensometer attachment 591, while FIG. 5C shows the eyeglasses 598 inserted into the lensometer attachment. These side-view illustrations also show that the apparatus 500 includes a second trigger switch 397 at the bottom side of the housing 502.

It will be noted from FIG. 5A that the apparatus 500 is binocular in design. In some binocular embodiments, both sides of the apparatus, addressing opposite eyes of a person using the apparatus, are designed to include optical elements similar to those illustrated in FIG. 2. In this way, measurements can be obtained for both eyes of a person using the apparatus at the same time. Embodiment apparatuses similar to that described in connection with FIGs. 5A-5C can simplify alignment of both eyes simultaneously with respective sides of the apparatus.

However, in the embodiment illustrated in FIGs. 5A-5C, one side of the apparatus 500 is configured to perform wavefront aberrometry measurements of the eye or eyeglass lens placed in front of the port 505, while the other side of the apparatus 500 is configured to have the same light transmission characteristics as the measurement-optical channel, but can otherwise be passive and see-through (i.e., open view). This can ensure that the user has a similar view through both eyes, instead of having the view of one eye brighter than the view of the other eye, for example. Thus, in order to perform both measurements on both eyes using the apparatus 500, the apparatus can be rotated 180° to address opposite eyes of a person using the apparatus 500, and opposite lenses of eyeglasses when used in lensometer mode, each eye or eyeglass in turn. Such open-view, binocular embodiments can permit the viewing conditions of both eyes to be similar to each other. This is in contrast to existing small wavefront aberrometers that are neither open view nor binocular, which makes the viewing conditions of the patient's two eyes different, which can negatively affect binocular subjective refraction (natural viewing).

FIG. 7 is a flow diagram illustrating a procedure 700 for determining a property of an eye. The property can include wavefront error produced by the eye, a refractive prescription for the eye, an accommodation range measurement, a presbyopia measurement, a phoropter measurement, and other measurements as described herein. Embodiment devices described herein, such as those described in connection with FIGs. 1-5C, may be used to perform the example procedure 700.

At 713a, a variable focal power is applied to light received from an eye, via a port of a housing configured to receive the eye, using a visual tunable lens. At 713b, light is passed from the eye along an optical path. At 713c, a wavefront of the light from the eye is measured, with the light being received via an optical path from the port of the housing.

At 713d, a property of the eye is determined based on the wavefront of the light from the eye. Further details regarding subject wavefront aberrometer procedures encompassed by procedure 700 are described hereinafter.

A further advantage of subject wavefront aberrometers described herein, in contrast to existing methods and systems, is that objective accommodation measurements can be obtained by acquiring wavefront measurements at any time during or between changes to the tunable lens settings, all while the patient views the same distant target through the tunable lens whose settings are changed as needed. In this manner, a very precise determination of accommodation can be obtained, which is not possible with existing methods and systems, even where both lens systems and wavefront aberrometers are both used in the same setting but as part of different systems.

In some embodiments of the subject wavefront aberrometers, no subjective feedback from the patient is even required for an accommodation measurement, because wavefront measurements are iteratively made while the tunable lens setting is changed until the wavefront measurements indicate that accommodation is no longer occurring. The accommodation amplitude measurement can be completed more rapidly since there is no need to wait for the patient's verbal responses. Patients that are asked to provide subjective feedback during an eye examination, such as an accommodation range examination, are often stressed about their feedback and even question their own final results because they are not sure whether their responses have been "correct." The objectivity that can be provided by an embodiment tunable lens and wavefront aberrometry combined system can eliminate the stress of these patients. The results can be more repeatable because they are not affected by a patient's anxiety regarding "correct" responses and because of the inherent precision of wavefront aberrometry. The accuracy of the measurements using embodiments described herein can also be more reliable because patient communication issues (e.g., with children, elderly patients, patients that do not speak the same language as a clinician, etc.). An example subject wavefront aberrometer method for determining accommodation using a combined tunable lens and wavefront aberrometry apparatus is further described hereinafter in connection with FIG. 6C.

FIG. 6A is a flow diagram illustrating in greater detail how objective refractive measurements may be obtained using subject wavefront aberrometer apparatus and methods, particularly by taking advantage of a visual tunable lens according to an objective refraction procedure 900c. At 955a, light is sent from an illumination light source, to an eye, along an optical path through a light source tunable lens and a visual tunable lens. Both the light source tunable lens and visual tunable lens are initially set to apply zero focal power. At 955b, light that is reflected or backscattered from the retina of the eye is passed through the visual tunable lens to a wavefront sensor.

At 955c, a wavefront of the light from the eye is measured. At 955d, the refractive error (e.g., spherical and astigmatic) of the eye is estimated based on the measured wavefront, together with focal power applied subsequently by the visual tunable lens and light source tunable lens. At 955e, appropriate focal powers (e.g. spherical and astigmatic) are applied by the visual tunable lens and light source tunable lens to negate an estimated refractive error of the eye to the greatest degree possible in view of the quality and available adjustments of the tunable lenses. At 955f, elements 955c, 955d, and 955e are repeated until an estimated refractive error of the eye is stable to within an acceptable level of variation (e.g., 0.25 dpt, 0.15 dpt, or 0.05 dpt).

FIG. 6B is a flow diagram illustrating in further detail how subjective refractive measurements can be obtained using embodiment apparatus and methods, according to a subjective refraction procedure 900d. At 957a, a visual tunable lens is set to negate refractive error of the eye of the user, where the refractive error is estimated from an objective refraction process such as that illustrated in FIG. 6A. At 957b, spherical and astigmatic power of the visual tunable lens are varied systematically (in line with standard subjective refraction practices), either automatically through a predefined method, or by manual input from the eye patient or an assistant.

At 957c, eye patient feedback is requested regarding comfort and visual acuity after each change of power of the visual tunable lens. At 957d, elements from 957b-c are repeated until an eyeglass prescription for the eye patient has been fully determined in line with standard subjective refraction procedures (e.g., using a phoropter). Accordingly, because subjective refraction as illustrated in example FIG. 6B may use objective results from example FIG. 6A as a starting point, refractive prescriptions and other properties determined by a determination module such as the module 120 illustrated in FIG. 1 or the determination and control module 220 illustrated in FIG. 2 can be based on both the wavefront aberrometry (objective results) and the tunable-lens-based phoroptry (subjective results) from the same apparatus.

FIG. 6C is a flow diagram illustrating an example accommodation procedure 900e that shows how embodiment devices and methods can be used to measure accommodation amplitude for evaluation of presbyopia. At 959a, a visual tunable lens is set to negate refractive error of a patient's eye as determined by subjective refraction. At 959b, the patient is requested to view through the apparatus toward a target with small text or symbols, such as a reduced Snellen chart, for example, placed at typical reading distance away from the eye, about 0.4 meters.

At 959c, minus optical power is added to the visual tunable lens gradually until the small text or symbols on the target become, and remain, blurred based on feedback from the patient. At 959d, accommodation amplitude of the patient's eye is determined by adding the total minus power of the visual tunable lens to the reciprocal of the distance of the target (about 1/0.4 m).

While patient feedback in combination with tunable lens adjustments alone may be used to determine accommodation range, a particular advantage of subject wavefront aberrometers described herein, including those with both a wavefront sensor and tunable lens in the same apparatus, is that accommodation may be measured in a more automated fashion by taking advantage of wavefront measurements in combination with tunable lens adjustments. As an example, objective and subjective refractive measurements may be performed first, as outlined in FIGs. 6A- 6B. This can provide final corrective prescription for the patient, initially without regard to accommodation range, and the visual tunable lens may be set to the final settings. Subsequently, the apparatus may measure an initial corrected wavefront with these tunable lens settings, and the apparatus may then change the tunable lens focal power very slowly in small steps, allowing for the patient's given eye under test to accommodate while still viewing the fixed target indicia.

At each lens adjustment step, after appropriate accommodation, an additional wavefront measurement can be automatically acquired by the apparatus, saved, and monitored by the determination module. After a sufficient number of steps in focal power, when the determination module eventually determines that the measured wavefront has deviated at least a minimum threshold from the initial corrected wavefront value (or otherwise determines from the wavefront measurements that the eye under test is no longer sufficiently accommodating), then the determination module may determine that a difference between the tunable lens focal power at the final optimized settings and the focal power at the point of maximum accommodation is the accommodation range of the patient's eye. As will be understood in view of this description, accommodation measurements such as those described above may also be performed according to binocular embodiments (subject wavefront aberrometers) on both eyes at the same time.

FIGs. 10A-10B are flow diagrams illustrating successive parts of a procedure 1000 for determining subjective refractive preferences of a patient using subject wavefront aberrometer apparatuses. It should be understood that the procedure illustrated in FIG. 6B for subjective refraction is a general procedure that can further include many different variations using embodiment apparatuses. In general, the procedure 1000 in FIGs. 10A-10B is a particular variation that includes iterative determination of coarse and fine subjective refractive preferences for a given eye and allows a patient to interact directly with an apparatus having interactive features to determine the subjective preferences. This can be done with smart, iterative control of visual-tunable-lens vision correction values using interactive patient feedback.

In some subject wavefront aberrometers, an optometrist, technician, or assistant asks the patient which correction settings for the visual tunable lens are subjectively better, iteratively, as refractive values of the visual tunable lens are changed, similar to the iterative procedure used in standard, optometrist-assisted phoroptry measurements. However, in the procedure 1000, the subject wavefront aberrometer apparatus requests that the patient turn the dial 356, which is set to control certain refractive values of the visual tunable lens, iteratively, over coarse and then fine ranges, and the device records the final settings made by the patient to refine subjective preferences. Each time the patient is asked by the device, through the speaker 362 illustrated in FIG. 3, to optimize a setting, the patient turns the dial 356 on the housing of the apparatus, while viewing a target such as a Snellen chart through the apparatus, until the patient is satisfied that he or she has adjusted the dial such that the visual tunable lens is set to the best value for visual acuity. Then, the apparatus automatically records the optimum tunable lens parameter found by the patient, as particularly described hereinafter. In other embodiments, the communication interface 360 in FIG. 3 may be used only to query the eye patient verbally and receive a voice-recognized verbal response from the patient, such as "one" or "two," regarding which subjective, refractive preference is better.

Another feature of the example procedure 1000 is that it illustrates how the orthogonal basis set, spherical equivalent power M, vertical Jackson cross cylinder J0, and oblique Jackson cross cylinder J45 can be set mutually independently by the apparatus. This is in contrast to other embodiments that use the standard clinical S, C, & A basis set referenced hereinabove in relation to FIG. 3, for example. It will be understood that an embodiment apparatus that has control over S, C, and A mutually independently may also control M, J0, and J45 mutually independently by a mathematical transformation.

In general, the procedure 1000 includes setting the visual tunable lens to the optimum settings determined from the objective refraction process using the wavefront aberrometer. An example procedure for determining objective refraction is described in connection with FIG. 6A. Thereafter in the procedure 1000, coarse subjective settings are determined. This is followed by setting the visual tunable lens to the optimum coarse subjective refractive value settings and then determining fine subjective refractive settings. The finer subjective refractive settings are used as the final subjective refractive preference values for the patient, and a refractive prescription may then be determined based on the fine subjective settings, for example. It should be understood that "setting the visual tunable lens," as used herein, can include setting one or more of a plurality of individual tunable lenses optically arranged in series, as described hereinabove in relation to FIG. 1.

In greater detail, in FIG. 10A at 1063, the visual tunable lens is set to optimum objective values for M, J0, and J45 (Mopt, J0opt, and J45opt, respectively) previously determined from objective refraction process based on wavefront aberrometry (see, e.g., FIG. 6A). These optimum objective values can be stored in the memory 470 illustrated in FIG. 4, and the lens settings can be made in response to commands from the processor 472 in FIG. 4, for example. Accordingly, at 1063a-c, M is set to Mopt, J0 is set to J0opt, and J45 is set to J45opt, respectively.

At 1065, coarse subjective settings Mopt', J0opt', and J45opt' are determined. In the procedure 1000, coarse subjective settings are determined in the following manner. At 1065a, the dial 356 is set to control the visual tunable lens such that, over a full range of motion of the dial available to the patient, M will vary over a range of Mopt +/- 0.5 dpt while J0 and J45 are maintained constant at J0opt and J45opt, respectively. At 1065b, the apparatus, via the speaker 362, instructs the patient to turn the dial 356 iteratively to optimize subjective visual acuity preference. During this adjustment, a full range of motion of the dial 356 only allows adjustment over the Mopt +/- 0.5 dpt range, such that the patient cannot deviate too far from the optimum objectively determined setting. It should be understood that the range of +/- 0.5 dpt for the coarse adjustment is an illustrative value, and this value may be changed and set in the apparatus based on further engineering, doctor or optometrist knowledge, machine learning , demographic factors, or other factors, as necessary. At 1065c, the apparatus then saves this value as the coarse subjective preference Mopt' and sets the visual tunable lens to this value.

At 1065d, the apparatus configures itself to control vertical Jackson cross cylinder J0 in response to a patient adjusting the dial 356. In particular, the apparatus sets itself to adjust J0 over a range of J0opt +/- 0.5 dpt as the dial 356 is adjusted over its full range. Meanwhile, the apparatus maintains the visual tunable lens at constant Mopt' and J45opt. At 1065e, the patient is requested, through the speaker 362, to turn the dial 356 iteratively to optimize J0 to an optimum coarse subjective preference value J0opt'. At 1065f, the apparatus then saves J0opt' and sets the visual tunable lens to this value.

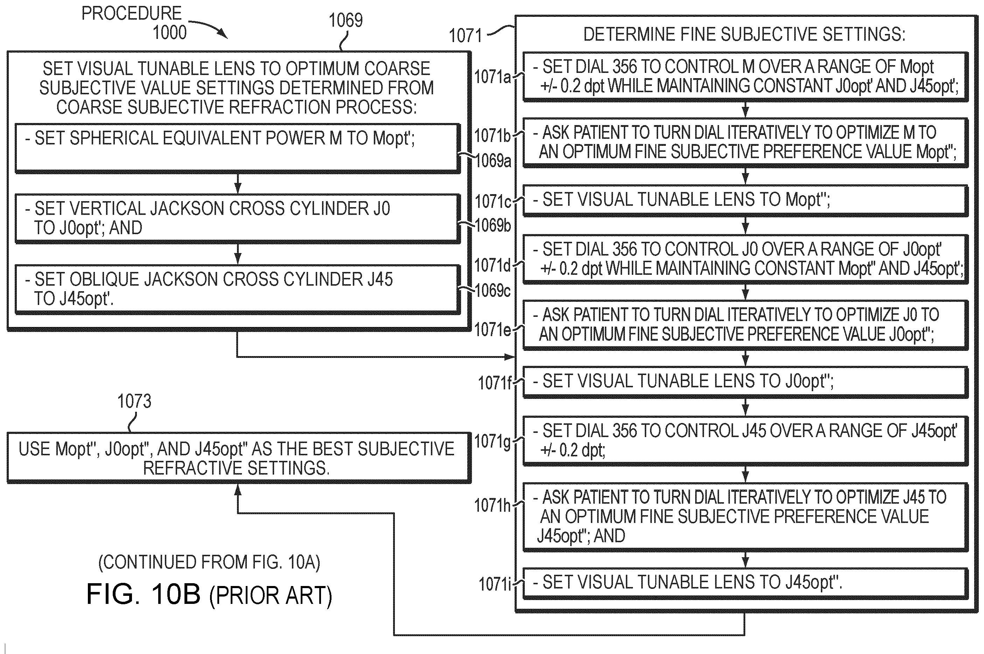

At 1065g, a similar procedure is carried out for the parameter J45. The apparatus sets itself to control J45 over a range of J45opt +/- 0.5 dpt as the patient turns the dial 356 over its full range, while maintaining constant values Mopt' and J0opt'. At 1065h, the apparatus asks the patient to turn the dial 356 iteratively to optimize visual acuity, and the patient finally settles on a preferred setting. At 1065i, the apparatus saves the setting as the optimum coarse subjective preference value of J45, namely J45opt'. With the coarse subjective refractive settings having been determined according to the patient's preferences, at 1067, the apparatus proceeds to determine the fine subjective settings, as illustrated in FIG. 10B, where the procedure 1000 is continued.

In FIG. 10B, in greater detail, at 1069, the apparatus sets the visual tunable lens to the coarse subjective settings determined at 1065 in FIG. 10A, if this has not already been done. Particularly at 1069a-c, the visual tunable lens set to Mopt', J0opt', and J45opt', respectively. At 1071, fine subjective settings are then determined in a manner similar to the manner used to determine the coarse subjective settings, except that the coarse subjective settings are used as the starting point instead of the objective settings. An illustrative, example fine range variation of +/- 0.2 dpt variation is used for each parameter. However, as noted above in relation to the coarse variation range, this fine variation range may be selected or set based on additional information or preferences.

At 1071a, the apparatus is set to respond to the patient's turning of dial 356 over its full range by controlling M correspondingly over a range of Mopt' ± 0.2 dpt while maintaining constant J0opt' and J45opt'. At 1071b, the patient is requested through the speaker to turn the dial iteratively to optimize visual acuity for the particular eye, OD or OS, that is under test. At 1071c, the fine subjective preference Mopt" is saved in memory, and the visual tunable lens is set to this value.

At 1071d, the apparatus sets itself to control J0 over a range of J0opt' +/- 0.2 dpt in response to the dial being changed over its full range, while still maintaining constant Mopt" and J45opt'. At 1071e, the apparatus asks the patient to turn the dial iteratively to optimize visual acuity. At 1071f, the apparatus records the value J0opt" and sets the visual tunable lens to this value. At 1071g, the apparatus configures itself to control J45 over a range of J45opt' +/- 0.2 dpt in response to the dial being turned over its full range. At 1071h, the patient is requested to turn the dial iteratively to optimize visual acuity. At 1071i, the apparatus records the optimum fine subjective preference value J45opt" and sets the visual tunable lens to this value.

At 1073, Mopt", J0opt", and J45opt" are then used as the best subjective refractive settings. These values may be set on the apparatus for a final confirmation from the patient that the settings are valid and acceptable. While not illustrated in FIGs. 10A-10B, the apparatus may optionally perform other functions at this point. For example, the apparatus may show the patient corrected and uncorrected views by changing the visual tunable lens, while speaking to the patient accordingly, similar to procedures followed by clinicians during traditional phoroptry. Furthermore, the apparatus may optionally give the patient a further opportunity to indicate that additional adjustments are preferred, either by pressing the trigger switch 397 illustrated in FIG. 3 or by the patient answering "yes" through the microphone 364 illustrated in FIG. 3, for example.

The procedure 1000 may also be repeated for each eye OD and OS in turn. Still further, the procedure 1000 may be modified such that coarse subjective testing is performed on each eye OD and OS in turn, followed by fine subjective testing on each eye in turn. Furthermore, it will be recognized by those skilled in the art of optometry that there are advantages in determining subjective refractive corrections of both eyes at the same time. As is known in the art, a patient's preferred correction for a given eye may differ depending on whether the other eye is looking through a correction lens, is uncorrected, or is blocked at the same time the given eye is evaluated. Accordingly, it will be recognized that, in the binocular arrangements described herein that allow for simultaneous simulated tunable lens correction for both eyes, the procedure 1000 may be modified such that subjective settings are tested for both eyes synchronously. For example, objective wavefront-based optimized tunable lens correction settings may be made for both eyes, followed by the patient or a clinician being directed to change a dial setting that simultaneously adjusts power or another parameter for both eyes together. In this way, a fine or coarse subjective setting may be determined.

Moreover, the procedure may be modified to include appropriate clinician involvement in any case where it is undesirable or impossible for a patient alone to make adjustments to optimize settings. The values Mopt", J0opt", and J45opt" may be reported at an interface similar to the reporting interface screen 354 illustrated in FIG. 3 and used to provide a refractive prescription. Furthermore, information determined from the procedure 1000, such as final, fine subjective refractive preferences, may be provided to a patient, clinician, manufacturer via any of the means described hereinabove or other known means.

It should be understood that the procedure 1000, in other embodiments, can be extended to successively finer adjustments and determinations of subjective refractive preference. Furthermore, higher-order refractive corrections may be determined in a manner similar to that illustrated in the procedure 1000, where a particular visual tunable lens used in the apparatus permits such adjustments. Those with skill in various types of multidimensional, iterative optimization, as well as those skilled in the art of optometry, will understand that "coarse" and "fine" subjective settings can further be determined even where the range of optimization (e.g., 0.5 dpt or 0.2 dpt) is the same for both coarse and fine determinations. This is because there is typically value in changing all the parameters to optimize values, followed by re-optimization of the same values, whether with the same or a smaller adjustment range available to the patient.

Moreover, wavefront aberrometry measurements may be interspersed with subjective measurements in any location within the procedure 1000 for a variety of purposes. As described hereinabove, subject wavefront aberrometers can perform adjustments of the variable focal power of the visual tunable lens or lenses iteratively in response to successive wavefront measurements to minimize wavefront errors of the light received from the eye or eyes. Wavefront measurements can be performed in a closed-loop fashion, or simply performed two or more times in between subjective measurements taking advantage of the tunable lens. One example includes obtaining an initial wavefront error measurement, setting a tunable lens to correct for the initial wavefront error, and then obtaining one or more secondary or subsequent wavefront measurements.

Performing wavefront measurements on eyes corrected by tunable lenses can allow higher-order corrections to be determined by wavefront aberrometry with greater accuracy that can be done with the same wavefront aberrometry instrument acting alone. As is known, it is useful to know higher-order corrections to apply to an eye for improved vision especially for low-light conditions and other specific cases. As such, subject wavefront aberrometers can enable wavefront measurement accuracy commensurate with a very expensive and precise wavefront aberrometer using a relatively much more inexpensive wavefront aberrometer. Use of a tunable lens in combination with a wavefront aberrometer in embodiments can enable more accurate measurement of higher-order aberrations, even with a relatively low-cost embodiment system, because the tunable lens can correct the primary low-order aberrations, thereby cancelling out the contributions of the low-order aberration (typically much larger), thus enabling better detection of the higher-order aberrations with better sensitivity and specificity.

Moreover, subject wavefront aberrometers combining tunable lenses with wavefront aberrometry can enable the subjective test (phoroptry) immediately after the objective wavefront aberrometry measurement in situ with the same handheld apparatus applied to the patient. This can provide better patient throughput and accuracy. Further, using subject wavefront aberrometers, objective measurements can be performed during the subjective phoroptry measurements. In this case, the objective measurements may be used in a situation in subjective phoroptry wherein the patient indicates that it is not clear which tunable lens setting of two or more choices given is better, for example.

Marks, Randall et al., "Adjustable adaptive compact fluidic phoropter with no mechanical translation of lenses," Optics Letters Vol. 35, No. 5, 739-741, March 1, 2010, is hereby incorporated herein by reference in its entirety.

The international Patent Cooperation Treaty (PCT) Applications published as WO 2015/003062 A1 and WO 2015/003086 A1 are hereby incorporated herein by reference in their entireties.

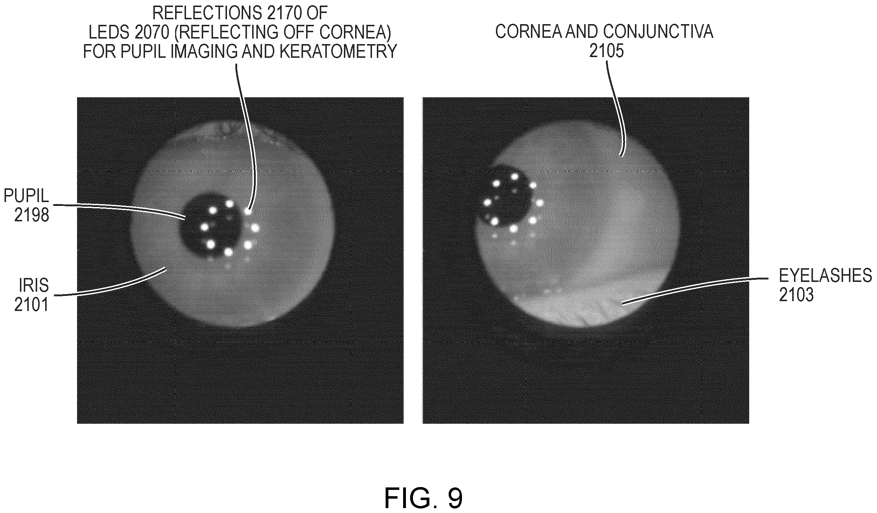

Embodiments of smart eyewear apparatus 3200 can include a pupil camera, such as that illustrated in FIG. 8 schematically showing a portable optical device 2008 as a generic reference to smart eyewear apparatus 3200, 3300. Smart eyewear memory 3250c may store corresponding executable code configured to assist in aligning, or in maintaining alignment of, one or more optical elements of the portable optical device/smart eyewear apparatus 3200 with a pupil of an eye of the end-user (wearer of the smart eyewear). Example drawings that illustrate aspects of such a pupil camera and alignment procedure include FIGs. 9, 11-13, for non-limiting example. In the case of smart eyewear apparatus 3200, use of the pupil camera for alignment is in addition to measuring pupil size and for iris biometric imaging as previously described.

There are several alignment indicators that can be provided in executable code such as software that enable alignment including use of: (i) a pupil tracking algorithm to identify and display the location of the pupil on the display screen, (ii) an orientation detection algorithm that evaluates the intensity of the individual LEDs and determines if there are differences above a threshold which will indicate whether the device/smart glasses is tilted or yawed with respect to the user's eye (particularly helpful since misalignments between the optics of the device and the user's eye can induce astigmatism in the measurements), (iii) a user-device distance algorithm which can include using autofocus algorithms, factory calibration files, or machine learning on ocular and facial features at various distances, which will estimate the distance from the eye to the device/smart eyewear apparatus 3200.

Maintaining the alignment can include maintaining the alignment for a sufficient time for the portable optical device 2008/smart eyewear apparatus 3200 to acquire a temporal sequence of wavefront aberrometry measurements of the eye. Furthermore, maintaining the alignment can be for a sufficient time to obtain a subjective refraction preference of the end-user while the end-user views one or more external target indicia through at least one visual tunable optical element of the portable optical device, such as a tunable optical lens 2072, 3216, 3218, as illustrated in FIGs. 8, 17, and 1, for example. Maintaining the alignment can further be for at least three seconds, five seconds, 10 seconds, 20 seconds, or 30 seconds, and for obtaining subjective refraction preferences of the end-user, maintaining alignment can be 1 - 5 minutes for non-limiting example.

As part of maintaining alignment, the device 2008/smart eyewear apparatus 3200 can track the position of the pupil continuously during the wavefront aberrometry measurement, the orientation of the device with respect to the user's eye, and the distance from eye to device's optics. If the device or the software operating it (on the device or in the cloud or at the edge) detects a momentary misalignment then the software can be configured to inform the operator or the user (or the motors) through the user interface and can provide indications on how to readjust the alignment.

The portable optical device 2008/smart eyewear apparatus 3200 can include one or more manual adjustments that are configured to permit the end-user, or an operator, to adjust alignment of the one or more optical elements of the portable optical device with an eye of the end-user. Such manual adjustments are illustrated in FIGs. 18B, and 19 for example. Alternatively, the corresponding executable code can be configured to cause one or more electric electromechanical actuators, such as motors, to adjust or maintain the alignment, instead of manual adjustments of the lead screw and knob, for non-limiting example.

The portable optical device 2008/smart eyewear apparatus 3200 can be configured for self-operation by the end-user with or without an assisting operator or technician.

The portable optical device 2008 can include a wavefront aberrometer, such as the wavefront sensor 116 described in connection with FIG. 1. The portable optical device 2008/smart eyewear apparatus 3200 can determine an optical property of an eye of the patient-user, such as refractive error or an initial refractive prescription, based on data from the wavefront aberration. This may be done, for example, via the execution of the executable code, which can perform, with with proper configuration that will be understood in view of this disclosure, all of the functions that are described for operators and physicians or other clinicians herein. In the case of the smart eyewear apparatus 3200, determining an optical property of the wearer-user's eye includes determining refractive power per depth of target viewed and custom calibration of the smart eyewear to this user as previously discussed in FIGs. 17-22 .

Determining the optical property based on data from the wavefront aberrometer can include fundus imaging or corneal imaging based on the data from the wavefront aberrometer. The optical path of the FIG. 1 or 2 (QuickSee^{™}) apparatus can be modified to include additional beamsplitters, diffusers, LEDs, as described at paragraphs 20-21, 25, 31-32 and 52, and FIGs. 1A / 1B, 1G, and 1K of PCT Pat. App. Pub. No. WO2020/010138 (hereinafter "Johns Hopkins publication," which is hereby incorporated herein by reference in its entirety). This will enhance QuickSee^{™} Plus optics and hardware to enable a wider measurement range and pupil imaging. Specifically, the Shack-Hartmann lenslet array employed in the QuickSee^{™} Plus optical channel can be replaced by a diffuser, with a predefined or random speckle pattern (sometimes referred to as a caustic pattern), to perform diffuser-based wavefront aberrometry. The diffuser's caustic or speckle pattern is unique and enables registration of large displacements, which enables a larger measurable range (in terms of diopters) than other wavefront aberrometry approaches that use a pinhole or lenslet-array based wavefront sensor.

By incorporating different wavelength (visible color such as Blue, Green, Red) LEDs into QuickSee^{™} Plus's optical channel, and appropriate beamsplitters, and an image sensor capable of detecting the visible color LEDs (e.g., RGB sensor), QuickSee^{™} Plus can perform computational lightfield ophthalmoscopy to image the retina, cornea, or lens. By simultaneously, or at almost simultaneously time intervals such as 0.1 seconds, 1 seconds, or less than 1 minute, or in a sequential manner, the light from the visible colored LEDs can be reflected by the beamsplitters specific to that wavelength of light, and then directed into the patient's eye. This will allow imaging of the fundus, which includes the retina, optic disc, macula, fovea, and / or the posterior pole. By using optical lens, or the tunable lens, in front of the specific LED or at the port closest to the patient's eye, a different visible colored LED (e.g., green) can be focused (the beam sizes can be modulated) to illuminate different portions of the eye, for instance, the entire anterior segment of the patient's eye to inspect the cornea. As another example, the visible color LED with the narrowest beam can be used for wavefront aberrometry after being reflected by the appropriate beamsplitter and being directed into the eye, with the return light being directed to the wavefront sensor. The light reflected and / or remitted from the patient's eye are emerging wavefronts that may bounce off of an appropriate beamsplitter to the wavefront sensor having a multi-channel image sensor (e.g., RGB sensor), which can capture the multiple caustic or speckle patterns. Multiplexing these different colored caustic or speckle patterns enables simultaneous or nearly simultaneous or sequential wavefront aberrometry measurements and high-quality images of the anterior and / or posterior segment of the eye in a synergistic manner.

The QuickSee^{™} Plus software / firmware function can be modified to include additional beamsplitters, diffusers, LEDs, as described at paragraphs 23-24, 25, 27-28, 37-41, and FIGs. 1C, 1E, 1F, 1H, 1I, 1J of the Johns Hopkins publication. This will enhance QuickSee^{™} Plus software / firmware to enable a wider measurement range and pupil imaging. Specifically, the image registration can be performed on the caustic of speckle images in comparison to a reference image. This process can utilize one or more well-known non-rigid image registration algorithms such as Demon's algorithm (including hyperparameter tuning), three-dimensional grid search for optimal parameters, a quantity of algorithm iterations, threshold-based approaches examining the reference image and test image for correlations, or determining a displacement matrix between pixels in the reference image and the test image. Machine learning / deep learning approaches can be employed to perform image registration and Zernike polynomial fitting. (1+1) Evolutionary optimization may also be performed.

The portable optical device 2008/smart eyewear apparatus 3200 can further include one or more visual tunable optical elements, and include a form or design that enables the end-user to have an open view through the one or more visual tunable optical elements and through the portable optical device . The corresponding executable code may be configured to determine an objective refraction and subjective refractive preference of the end-user, based on the open view of the patient-user.

The corresponding executable code can be configured to stream wavefront aberrometry data from the portable optical device/smart eyewear apparatus 3200 to a physician via audiovisual means, other network communication means, and the like, enabling the physician to generate a refractive correction or an eyeglass or contact lens prescription. The code can also be configured to generate keratometry data for an eye of the patient-user using the portable optical device. The keratometry data can be based on pupil imaging data from a pupil camera on the portable optical device 2008/smart eyewear apparatus 3200, such as the pupil camera 2088 illustrated in FIG. 8. The portable optical device 2008, 3200 can be of a binocular or monocular design, and can provide the patient-user an open view toward target indicia that are external to and spaced away from the portable optical device.

Specific example keratometry data that can be provided include the Horizontal axis (H) of the circular or elliptical pattern, the Vertical axis (V) of the circular or elliptical pattern, 'Avg' the mean of H and V, and 'Cylinder' the power of the corneal astigmatism.

In embodiments of the present invention, the corresponding executable code can be further configured to determine the degree of alignment based on a pupil image of the eye from pupil camera 2088 on the device, such as illustrated in FIG. 8. The executable code can be cloud-based software or code that is implemented within the portable optical device 2008/smart eyewear apparatus 3200, such as firmware or software. The code can be configured to process a wavefront image of the eye, where the wavefront imaged is received from the portable optical device 2008/smart eyewear apparatus 3200. Wavefront image processing has been described above in connection with FIGs. 1- 7, 10A, and 10B. As demonstrated above, the corresponding executable code is or may be configured to enable generation of a diagnosis of eye health of the patient-user. The diagnosis can include a refractive prescription for eyeglasses or contact lenses, but the diagnosis can also include retinal imaging based on a wavefront image of the eye acquired by the portable optical device 2008, 3200. The diagnosis can include presentation or use in diagnosis of keratometry measurements based on one or more images from the pupil camera 2088. Among the functions of enabling a diagnosis of eye health of the end-user, the code is configured to determine a subjective refractive preference of the patient-user during calibration of the smart eyewear apparatus 3200 the patient-user having an open view through the portable optical device2008, 3200. As mentioned previously, the code can be further configured to generate a contact lens prescription for the patient-user based on the subjective refractive preference and on the keratometry.

For wavefront aberrometry measurements to be of enhanced accuracy, the light source must pass through the patient-user's pupil, which can be 1.5 mm to 8 mm on average, during the initial measurement and during the subjective refraction and vision correction portion of device 2008, 3200 use and calibration. To achieve this level of precision over a prolonged exam period, it is advantageous to have: (1) a pupil camera to understand if the position of the optics relative to the pupil is correct and allows the wavefront aberrometry light source to pass through the pupil; (2) the position of optics of the device be adjustable relative to the end-user's pupil, either manually by the operator or end-user, or automatically (mechcanized and automated) by the remote physician/technician or artificial intelligence; (3) the device must be stably mounted to the user's pupil and face via a head or shoulder mount or other suitable means , which enables the measurements to be taken and visual acuity and subject refraction preference to be maintained even while the user looks around, thus ensuring a natural viewing experience to evaluate the corrective prescription. These and other advantages of embodiments of the present invention are made evident by the disclosure herein.

Turning to FIG. 8, a schematic diagram illustrates a portable optical device 2008/smart eyewear apparatus 3200, 3300.. The optical diagram illustrates an open-view wavefront aberration camera, pupil camera, and keratometry. There are two sources of illumination, including a laser diode 2080 that is used to illuminate a point of light on the retina of the eye 1754, and another, namely light emitting diodes 2070, that are mounted on an eye cup of the device 2008 and used to illuminate the cornea and the pupil of the eye 1754. By ensuring that the two illumination sources are sufficiently separated in wavelength, appropriate beam splitters 2074, 2076, and 2078 can be chosen to ensure that the light reflecting off of the retina is sent to the camera 2084 that performs wavefront aberrometry, while light reflecting off the cornea is sent to a pupil camera 2088. The device also includes focusing optics 2090 for focusing light into the pupil camera 2088, as well as focusing optics 2086 for focusing light from the retina onto the wavefront aberrometer camera 2084.

The laser diode 2080 produces light 2082 that is inbound laser light into the eye, which travels through the beam splitters and through a tunable optics 2072 into the retina. Laser light that is reflected off of the retina, 2094, enters the wavefront camera 2084. Light 2092 from the LEDs 2070, which is reflected off of the cornea, enters the focusing optics 2090 and enters the pupil camera 2088. The device 2008 features both the tunable optics 2072 and an open view, in part provided by a transparent window 2096, which allows the eye 1754 to see through the portable optical device 2008 to external target indicia 252 that are separated from the housing of the portable optical device 2008/smart eyewear apparatus 3200, 3300. In this manner, the eye 1754 is permitted to be relaxed and substantially accommodated for measurements.

FIG. 9 includes two images of a human pupil 2198 that is imaged with a portable optical device, such as the device 2008 of FIG. 8, having eight LEDs 2070 in a circular pattern. Similar images can be taken with other numbers of LEDs, such as four LEDs in a square pattern, for example. In the images, the pupil 2198, iris 2101, eyelashes 2103, and cornea and conjunctiva 2105 are clearly visible and identifiable. The reflections 2170 of the LEDs 2070 reflecting off of the cornea can be used for pupil imaging, iris imaging (for pupil sizing and biometrics), and keratometry.

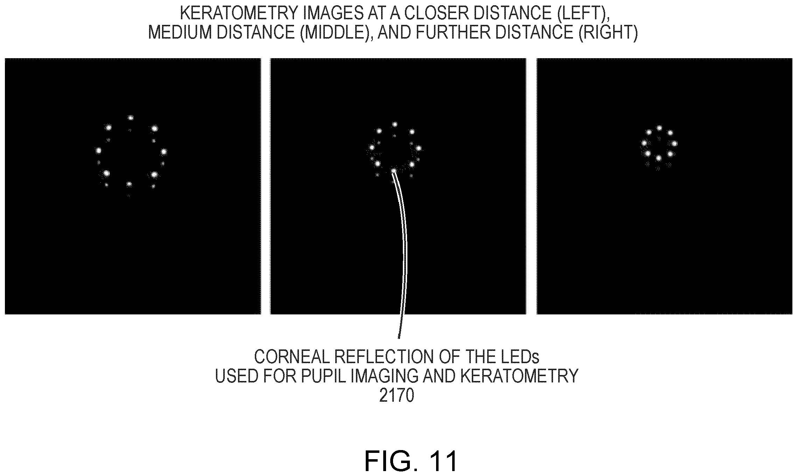

FIG. 11 includes a series of three keratometry images at a closer distance (left), medium distance (middle), and further distance (right). Keratometry data can be derived from the pupil camera images by processing (e.g., thresholding) the image. In this example, all pixel values were set under a threshold to zero, which results in extraction of the image of the LED light sources, since they are the brightest features in the images. The distance from the LEDs to the surface of the eye can be determined by various methods, including direct measurements with distance sensors e.g., physical ruler, optic or acoustic-based sensors, or through image processing of the pupil camera image. The position of the LEDs on the pupil camera image, actual physical positions of the LEDs, and the estimated distance of the LEDs from the surface of the eye, can all be used to determine the curvature of the cornea (keratometry values) using standard formulas. In this manner, by including a pupil camera on devices that are used in embodiments, keratometry data may be obtained in order to inform contact lens prescriptions.

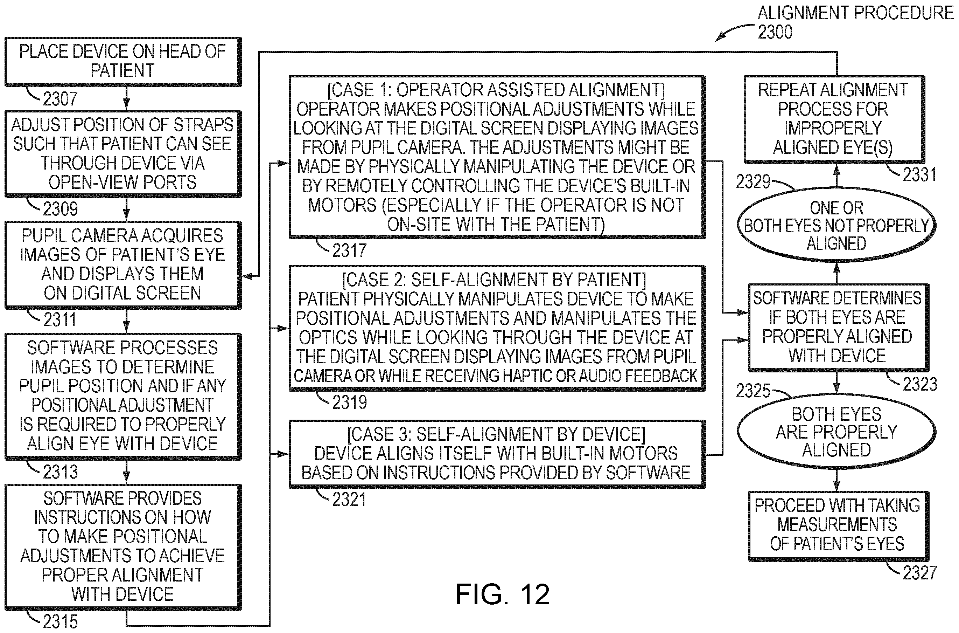

FIG. 12 is a flow diagram illustrating how alignment of device optics and patient-user pupils may be performed according to various embodiments. In particular, FIG. 12 shows an alignment procedure 2300. In the procedure, at 2307, the device 2008, 3200, 3300 is placed on the head and face of the patient or end-user (namely wearer of the smart eyewear). Nonetheless, it should be understood that similar procedures can be performed for handheld, shoulder-mounted, and stand mounted portable optical devices. However, head-mounted, shoulder-mounted, and tripod-mounted or other stand-mounted devices are preferred for longevity of alignment stability for best alignment and measurement results according to embodiments of subject wavefront aberrometers.

At 2309, a position of straps, such as the head strap and the like , , is adjusted such that the patient/smart eyewear user can see through the device via open-view ports. At 2311, a pupil camera acquires images of the patient's eye and displays them on a digital screen, such as the digital screen of the devices described in connection with FIGs. 1-7, and 10A-10B.

At 2313, software processes the pupil images to determine pupil position and whether any positional adjustment is required to align the eye with the device properly. At 2315, the software provides instructions on how to make positional adjustments to achieve proper alignment with the device.

Then, in a first case of operator-assisted alignment, at 2317, an operator or technician makes positional adjustments while looking at the digital screen displaying images from the pupil camera. The adjustments might be made by physically manipulating the device or by remotely controlling the devices built-in motors, especially if the operator is not on-site with the patient/end-user. In a second case, at 2319, a case of self-alignment by the patient/end-user, the patient-user physically manipulates the device to make positional adjustments and manipulates the optics while looking through the device at the digital screen displaying images from the pupil camera, or while receiving haptic or audio feedback, either from the device itself, or from a tablet or other secondary device. In a third case, at 2321, the device performs self-alignment by using built-in motors, based on instructions provided by corresponding software. At 2323, for the operator-assisted alignment and self-alignment, the software determines if both eyes are properly aligned with the device. At 2325, the software may determine that both eyes are properly aligned, in which case, at 2327, the device proceeds with taking measurements of the patient's/end-user's eyes. However, if the software determines at 2323 that both eyes are not properly aligned with the device, and more particularly, if one or both eyes are not properly aligned at 2329, then at 2331, the alignment process is repeated for the improperly aligned eye or eyes.

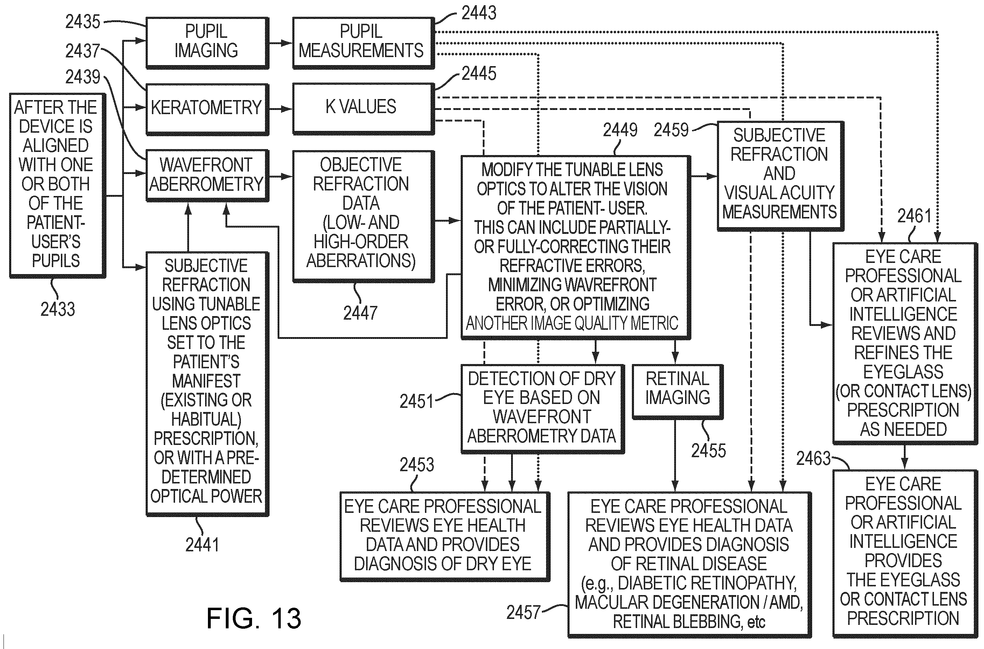

FIG. 13 is a flow diagram illustrating a process to obtain various eye health parameters from a portable optical device according to embodiments. Some or all of these eye health parameters can be measured simultaneously, or in feedback loops to iterate and refine the measurements. Some or all of these eye health parameters can be combined, in addition to being combined with other personal demographic or health history data, in order to assist an eye care professional or an artificial intelligence routine that is part of corresponding executable code in various embodiments, to provide diagnosis and prescription for eye health conditions of the patient-user (wearer of the smart eyewear apparatus 3200, 3300).

At 2433, after the device is aligned with one or both of the patient-user's pupils, four different processes of measurement may be obtained, either in sequence, or in some cases simultaneously, as will be understood by those of skill in the art in view of this disclosure. At 2435, pupil imaging can be performed, as previously described . At 2437, keratometry measurements can be obtained, for example based on the pupil imaging at 2435. At 2439, wavefront aberrometry can be performed. At 2441, subjective refraction measurements, using tunable lens optics set to the patients manifest (existing or habitual) prescription, or with a predetermined optical power, can be performed. The subjective refraction 2441 can be used as an input or precursor to wavefront aberration 2439. However, wavefront aberrometry 2439 (objective refraction) can also be performed as a starting point to obtain the subjective refraction 2441. The wavefront aberrometry can be used at 2447 to obtain objective refraction data, with both low- and high-order aberrations. At 2449, the tunable lens optics can be modified to alter the vision of the patient-user (wearer of the smart eyewear apparatus 3200, 3300). This can include partially or fully correcting the patient-user's refractive errors, minimizing wavefront error, or optimizing another image quality metric. At 2451, detection of dry eye based on wavefront aberrometry data may be performed. Then at 2453, an eye care professional can review eye health data and provide a diagnosis of dry eye if needed.

Furthermore, after the modification of tunable lens optics at 2449, retinal imaging 2455 can be performed. At 2457, an eye care professional can review by healthcare data and provide diagnosis of retinal disease based on the retinal imaging 2455. The retinal disease can include, for example, diabetic retinopathy, macular degeneration/AMD, retinal blending, etc. At 2459, subjective refraction and visual acuity measurements may also be performed, for example after minimizing wavefront error using the tunable lens settings at 2449, for example. The subjective refraction and visual acuity measurements may be used to inform an eye care professional, or artificial intelligence, at 2461, in order to refine an eyeglasses or contact lens prescription as needed. At 2463, the eye care professional or artificial intelligence can provide the eyeglasses or contact lens prescription to the patient-user or to an optical supplier.

The pupil imaging at 2435 may be used to produce pupil measurements at 2443, which can inform the contact lens prescription obtained at 2461, for example. Furthermore, the pupil measurements 2443 can be used as part of the eye health data review at 2453, for non-limiting example.

The keratometry measurements at 2437 can be used to determine K values at 2445, which can also inform the dry eye, retinal disease, or eyeglasses or contact lens prescription determination.

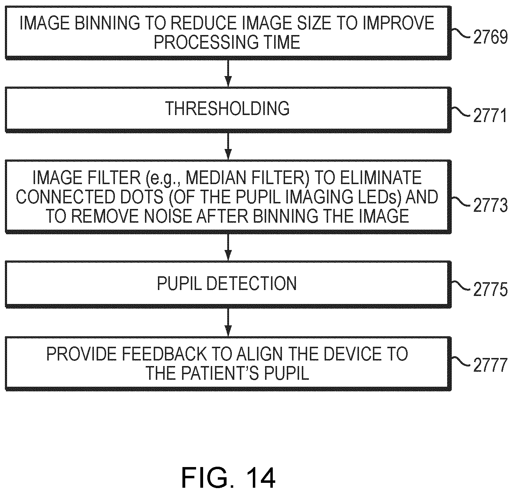

FIG. 14 is a flow diagram outlining how a pupil camera, and the images provided thereby, can be processed in order to determine a position of a pupil, which can be used to provide feedback on how to align the devices measurement optics to the end-user's pupil (pupil of the wearer of the smart eyewear apparatus 3200, 3300). At 2769, image binning is performed to reduce image size to improve processing time. At 2771, thresholding is performed, as previously noted. At 2773, image filtering is performed (e.g., via a median filter) to eliminate connected dots (of the pupil imaging LEDs) and to remove noise after binning the image. At 2775, pupil detection is performed. Finally, at 2777, feedback is provided to align the device to the patient's/end-user's pupil. At that time, the feedback may be automated via motorized actuators that the corresponding executable code automatically commands to move horizontal and vertical rails of the optic carriage to the correct position, or the feedback may be to the patient-user, or to an operator, such that manual positioning may be performed, such as using the lead screw and knob for horizontal and vertical adjustments .

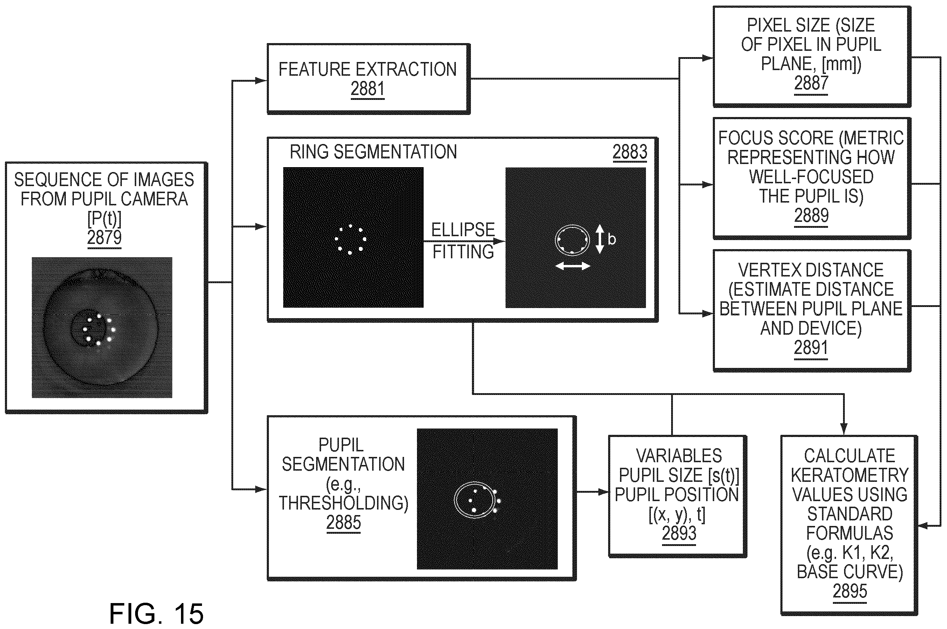

FIG. 15 is a flow diagram outlining how a sequence of images or plurality of unordered images (generally multiple images) captured by a pupil camera can be processed to obtain keratometry values of an eye in various embodiments. At 2879, a sequence of images from a pupil camera [P(t)] is obtained. At 2881, feature extraction is performed. At 2883, ring segmentation is performed, using, for example, ellipse fitting. At 2885, pupil segmentation is performed, such as by thresholding, as previously described.

Based on the feature extraction 2881, pixel size 2887, focus score 2889, and vertex distance 2891 can be obtained. Pixel size is the size of pixel in the pupil plane, in millimeters. The focus score is a metric representing how well focused the pupil is. The vertex distance is an estimate of distance between the pupil plane and the device. At 2883, based on the pupil segmentation 2885, the variables pupil size [s(t)] and pupil position [(x, y), t] can be obtained. Finally, based on the variables of pupil size and pupil position, the ring segmentation, and the pixel size, focus score, and vertex distance, at 2895, keratometry values can be calculated using standard formulas, such as K1, K2, and base curve.

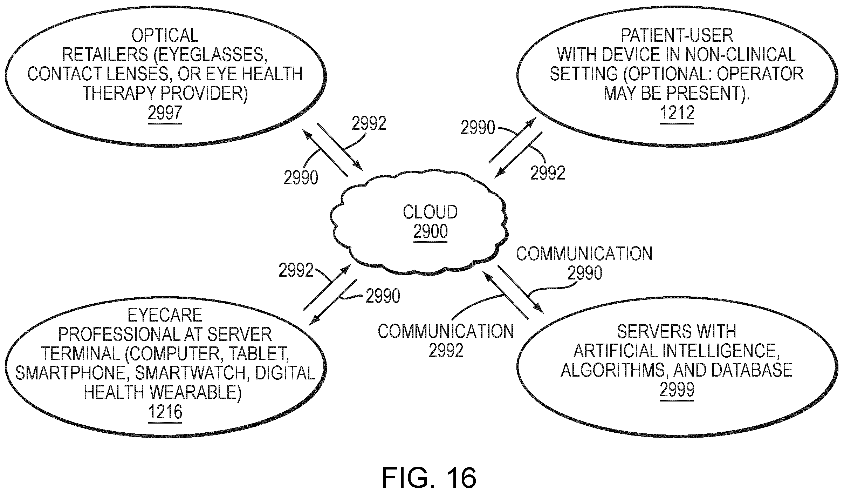

FIG. 16 is a schematic diagram illustrating a cloud environment 2900 showing potential interconnectedness of different components of a cloud ecosystem and how eye health data can be shared between a patient-user (wearer of smart eyewear apparatus 3200, 3300) and an eye care professional and or artificial intelligence in addition to use of the eye health data measurements for calibration of the smart eyewear apparatus 3200, 3300. FIG. 16 also schematically illustrates how subsequent or consequential therapeutic output, such as a prescription or diagnosis, can be relayed to a therapy provider, all according to alternative or extended embodiments further described herein in connection with the other drawings.

The cloud 2900 manages transfer of information between entities via wired or wireless communication 2990 to the entities or wired or wireless communication 2992 from the entities. The data can then be sent to servers to be further analyzed and stored in a database. The database can then be accessed by an authorized eye care professional from any Internet connected device, such as a computer, tablet, smart phone, etc. Alternatively, the patient-user's data taken by the hardware (subject wavefront aberrometer, smart eyewear apparatus 3200, 3300) can be uploaded by the end-user/wearer directly to the eye care professional from any Internet connected device, such as a computer, tablet, smart phone, etc. Known or common techniques and protocols for such data transmission, upload, and database storage are employed.

In particular in FIG. 16, an optical retailer 2997, which can provide eyeglasses, contact lenses, or eye health therapy, for example, can communicate through the cloud with an eye care professional 1216, which can provide a prescription or therapy order, for example. Alternatively, the optical retailer 2997 can receive a prescription from artificial intelligence housed on a server 2999 that determines a prescription or a diagnosis based on data received from the device 3200, 3300 that is used to measure a patient-user 1212. Still further, the optical retailer 2997 can receive a prescription, for example, directly from the device that is with the patient-user 1212 at the time of eye measurements , via the device having Wi-Fi connectivity with the cloud, or a wired connection to the cloud, for example. Still further, artificial intelligence that is capable of making a diagnosis or prescription may be present directly on the device 3200, 3300 or related accessory(ies) that is with the patient-user 1212 and may provide a prescription to the patient-user 1212, for example. The patient-user 1212 may then order eyeglasses, contact lenses, or request therapy from the optical retailer 2997 via a wired or wireless connection, for example.

In various embodiments, the eye care professional 1216 and a server terminal, which can include a computer, tablet, smart phone, Smart watch, digital health wearable, etc., can be enabled by the corresponding executable code at the location of the patient-user 1212 to participate in an eye examination of the patient-user 1212 using the device 3200, 3300 and/or related accessory(ies). This can be performed via the device directly streaming video or other data to the eye care professional 1216, or via a tablet or other connected secondary device, providing a link for audiovisual communication or other data connection between the eye care professional and either the patient-user or the portable optical device 3200, 3300 that is with the patient-user 1212.

In still other variations of embodiments, the device 3200, 3300 that is with the patient-user 1212 may communicate directly with a server 2999 that runs a portion of corresponding executable code that assists with: (a) alignment between the patient-user and the device, (b) diagnosis based on data from the device, or (c) prescription based on data from the device, for non-limiting example. Still further, the server 2999 with artificial intelligence can perform other functions of the operator or the eye care professional 1216 in order to interact with the patient-user 1212, the device that measures the patient-user, or a peripheral device such as a tablet, mobile device, and the like . In this manner, it will be understood that a wide variety of embodiments can be performed consistent with the claims and particular embodiments described herein.

The teachings of all patents, published applications and references cited herein are incorporated by reference in their entirety.

While example embodiments have been particularly shown and described, it will be understood by those skilled in the art that various changes in form and details may be made therein without departing from the scope of the embodiments encompassed by the appended claims.

### Items

1. A method of improving visual experience from open view smart eyewear, comprising:
   objectively measuring refraction of a user viewing through an open view smart eyewear, said objective measuring resulting in refraction measurements of the user; and
   using the resulting refraction measurements, either (i) updating tunable optics in accordance with detected optical needs of the user, the tunable optics being operatively associated with the open view smart eyewear, or (ii) updating content displayed to the user by the open view smart eyewear.
2. The method of item 1, wherein the smart eyewear is any of: an augmented reality headset, a virtual reality headset, a mixed reality headset, or portable head mounted open view apparatus.
3. The method of item 1, wherein objectively measuring refraction includes applying a wavefront aberrometer module to visual performance of the user's eyes while the user is wearing and viewing through the smart eyewear.
4. The method of item 3, wherein the wavefront aberrometer module is removably coupled to the smart eyewear.
5. The method of item 3, wherein the wavefront aberrometer module is a built-in component of the smart eyewear.
6. The method of item 3, wherein updating tunable optics updates tunable optical elements of the wavefront aberrometer module.
7. The method of item 3, wherein updating tunable optics updates tunable optical elements of the smart eyewear.
8. The method of item 3, wherein the wavefront aberrometer module further determines refraction of each eye of the user at multiple view depths, and calibrates the tunable optics to response of the user's eye to different lighting conditions, depths of view, or target contrast.
9. The method of item 8, further comprising a pupil camera capturing change in size of pupil of the user's eye, and therefrom providing a measure of pupil response to different light intensities and a measure of impact of pupil size on high order aberrations to calibrate the tunable optics.
10. The method of item 3, wherein the wavefront aberrometer module further includes dynamic wavefront sensing.
11. The method of item 3, wherein the wavefront aberrometer module includes a wavefront sensor, the tunable optics being configured to work in combination with or to receive input from the wavefront sensor such that the tunable optics provide any wavefront error cancellation in a feedback loop responsive to iterative wavefront measurements.
12. The method of item 3, wherein the tunable optics have an initial setting and the wavefront aberrometer module iteratively adjusts the tunable optics in response to detected optical needs of the user.
13. The method of item 12, wherein the initial setting is representative of a subjectively determined measurement of refractive error based on user input.
14. The method of item 12, wherein the initial setting is representative of an objective measurement of refractive error of an eye of the user as generated by the wavefront aberrometer module.
15. The method of item 1, further comprising capturing iris biometric data from the user as biometric identification of the user.
16. The method of item 15, wherein capturing iris biometric data includes employing a sensor or camera, operation of the sensor or camera further: (i) senses the user changing eye fixation from something far to something near; (ii) responsively measures accommodation amplitude or lag of the user's eye; and (iii) based on measurements of accommodation, enables calibration of the tunable optics to be customized to the user's vision performance.
17. The method of item 1, wherein the updating content displayed is in accordance with detected optical needs of the user.
18. A smart eyewear calibration device comprising:
   a wavefront aberrometer module configured to be operatively coupled to open view smart eyewear, the smart eyewear including a processing component,
   the wavefront aberrometer module supporting calibration of a tunable optical element in accordance with detected optical needs of a user viewing through the smart eyewear, by the wavefront aberrometer module being configured to: (a) objectively measure eye refraction of the user while wearing and viewing through the smart eyewear, and (b) use objective refraction measurements to update the tunable optical element or to update contents displayed to the user by the smart eyewear in a manner that improves visual experience of the user.
19. The calibration device of item 18, wherein the wavefront aberrometer module is configured to be removably coupled to the open view smart eyewear.
20. The calibration device of item 18, wherein the processing component is communicatively coupled to computer memory storing calibration parameters and eye refraction measurements from the wavefront aberrometer module, such that subsequent to calibration, the processing component automatically further adjusts the tunable optical element or further updates contents displayed to the user as a function of changed viewing conditions throughout use of the smart eyewear by the user.

## Claims

1. A method of improving visual experience from open view smart eyewear, comprising:
objectively measuring refraction of a user viewing through an open view smart eyewear, said objective measuring resulting in refraction measurements of the user; and
using the resulting refraction measurements, either (i) updating tunable optics in accordance with detected optical needs of the user, the tunable optics being operatively associated with the open view smart eyewear, or (ii) updating content displayed to the user by the open view smart eyewear.

2. A method as claimed in claim 1, wherein the smart eyewear is any of: an augmented reality headset, a virtual reality headset, a mixed reality headset, or a portable head mounted open view apparatus.

3. A method as claimed in any of the preceding claims, wherein objectively measuring refraction includes applying a wavefront aberrometer module to visual performance of the user's eyes while the user is wearing and viewing through the smart eyewear.

4. A method as claimed in claim 3, wherein the wavefront aberrometer module is removably coupled to the smart eyewear; or
wherein the wavefront aberrometer module is a built-in component of the smart eyewear.

5. A method as claimed in any of claims 3-4, wherein the updating tunable optics updates tunable optical elements of the wavefront aberrometer module; and/or
wherein the updating tunable optics updates tunable optical elements of the smart eyewear.

6. A method as claimed in any of claims 3-5, wherein the wavefront aberrometer module further determines refraction of each eye of the user at multiple view depths, and calibrates the tunable optics to response of the user's eye to different lighting conditions, depths of view, or target contrast.

7. A method as claimed in Claim 6, further comprising a pupil camera capturing change in size of pupil of the user's eye, and therefrom providing a measure of pupil response to different light intensities and a measure of impact of pupil size on high order aberrations to calibrate the tunable optics.

8. A method as claimed in any of claims 3-7, wherein the wavefront aberrometer module further includes dynamic wavefront sensing.

9. A method as claimed in any of claims 3-8, wherein the wavefront aberrometer module includes a wavefront sensor, the tunable optics being configured to work in combination with or to receive input from the wavefront sensor such that the tunable optics provide any wavefront error cancellation in a feedback loop responsive to iterative wavefront measurements.

10. A method as claimed in any of claims 3-9, wherein the tunable optics have an initial setting and the wavefront aberrometer module iteratively adjusts the tunable optics in response to detected optical needs of the user;
such as wherein the initial setting is representative of a subjectively determined measurement of refractive error based on user input; or wherein the initial setting is representative of an objective measurement of refractive error of an eye of the user as generated by the wavefront aberrometer module.

11. A method as claimed in any of the preceding claims, further comprising capturing iris biometric data from the user as biometric identification of the user;
such as wherein capturing iris biometric data includes:
employing a sensor or camera, operation of the sensor or camera further: (i) senses the user changing eye fixation from something far to something near; (ii) responsively measures accommodation amplitude or lag of the user's eye; and (iii) based on measurements of accommodation, enables calibration of the tunable optics to be customized to the user's vision performance.

12. A method as claimed in any of the preceding claims, wherein the updating content displayed is in accordance with detected optical needs of the user.

13. A smart eyewear calibration device comprising:
a wavefront aberrometer module configured to be operatively coupled to open view smart eyewear, the smart eyewear including a processing component,
the wavefront aberrometer module supporting calibration of a tunable optical element in accordance with detected optical needs of a user viewing through the smart eyewear, by the wavefront aberrometer module being configured to: (a) objectively measure eye refraction of the user while wearing and viewing through the smart eyewear, and (b) use objective refraction measurements to update the tunable optical element or to update contents displayed to the user by the smart eyewear in a manner that improves visual experience of the user.

14. The calibration device as claimed in claim 13, wherein the wavefront aberrometer module is configured to be removably coupled to the open view smart eyewear; and/or
wherein the processing component is communicatively coupled to computer memory storing calibration parameters and eye refraction measurements from the wavefront aberrometer module, such that subsequent to calibration, the processing component automatically further adjusts the tunable optical element or further updates contents displayed to the user as a function of changed viewing conditions throughout use of the smart eyewear by the user.

15. The calibration device as claimed in any of claims 13-14, wherein the device is arranged to carry out the method of any of claims 1-12.
